# EUROPEAN PATENT APPLICATION

(11) **EP 3 838 272 A2**
(43) Date of publication of application: **23.06.2021**
(21) Application number: 19823531.9
(22) Date of filing: 21.06.2019
(51) Int. Cl.: A61K 31/436, A61P 25/28, A23L 33/10

(54) **COMPOSITION FOR PREVENTION OR TREATMENT OF NEURODEGENERATIVE DISEASE**

(30) Priority: 21.06.2018 KR 20180071564
(71) Applicant: The Asan Foundation, Seoul 05505 (KR); University Of Ulsan Foundation For Industry Cooperation, Ulsan 44610 (KR)
(72) Inventor: KOH, Jae-Young, Seoul 06348 (KR); KIM, Ha Na, Seoul 05242 (KR); KIM, Tae-Youn, Seoul 05398 (KR); LEE, Huikyoung, Seoul 05395 (KR)
(74) Representative: Dehns
(86) International application number: PCT/KR2019/007556
(87) International publication number: WO 2019/245347

(57) **Abstract**

The present invention relates to a pharmaceutical composition for prevention or treatment of neurodegenerative disease, a composition employing amlexanox, which has been verified for safety through clinical trials. In addition, the present invention relates to a pharmaceutical composition for prevention or treatment of amyloid beta-caused cell damage, the composition employing a phosphodiesterase (PDE) inhibitor inclusive of amlexanox. The composition of the present invention can be advantageously and safely used for ameliorating or treating amyloid beta-caused neurodegenerative disease and cell damage, without worry about side effects.

## Description

### [Technical Field]

The present invention relates to a composition for preventing or treating a neurodegenerative disease, and more particularly, to a composition for preventing or treating a neurodegenerative disease and amyloid beta-caused cell damage, which contains a phosphodiesterase (PDE) inhibitor or a pharmacologically acceptable salt thereof as an active ingredient, which is able to be effectively used in prevention or treatment of a neurodegenerative disease such as Alzheimer's disease, Parkinson's disease or Lou Gehrig's disease.

This application claims priority to and the benefit of Korean Patent Application No. 10-2018-0071564, filed on June 21, 2018, and Korean Patent Application No. 10-2019-0074410, filed on June 21, 2019, the disclosures of which are incorporated herein by reference in their entirety.

### [Background Art]

Neurodegenerative diseases are associated with symptoms generated in degeneration, function loss or death of neurons. Since these are usually progressive, their results are very destructive. Patients with neurodegenerative diseases experience extreme degeneration in cognitive or motor function. Accordingly, the patients do not have the quality or expectation of life at all.

Research on the mechanism of Alzheimer's disease is mostly based on the amyloid theory, and based on this, studies have been mainly focused on APP, BACE and Presenilin genes, the interaction and regulation with other factors in cells, and the generation and degradation factors and signaling. Therefore, even when a single compound was prepared by synthesis, a target substance was focused on "BACE1" and "the inhibition of amyloid beta fibril formation."

However, recently, it was verified that compounds such as an amyloid beta aggregation inhibitor (Alzhemed) and a γ-secretase inhibitor (Flurizan) do not have a substantial effect on early Alzheimer-type dementia patients, and ACC001 (amyloid vaccine) under development has not been commercialized because of the side effects generated by an immune response.

Meanwhile, among neurodegenerative diseases, amyotrophic lateral sclerosis (ALS) widely known as Lou Gehrig's disease is associated with degenerative death of motor neurons of the central nervous system, and a disease which makes a patient paralyzed, and at the end, there is a symptom of respiratory difficulty, leading to death within 2 to 5 years. Among all types of ALS, sporadic ALS (sALS) accounts for 90 to 95%, familial ALS (fALS) accounts for 5 to 10%, and among the familial ALS, approximately 20% has been found to be caused by a mutant SOD-1 protein. Although the pathological mechanism of ALS was not clearly known, it has been reported that ALS is caused by a genetic factor or a combination of oxidative damage, abnormal protein accumulation, excitotoxicity, mitochondrial damage and inflammation. The only drug developed until now as a therapeutic for Lou Gehrig's disease is riluzole, which is in clinical trials. However, this drug only slows down the progression of Lou Gehrig's disease, and is not a fundamental solution. Therefore, the existing drug also has limitations.

In neurodegenerative diseases such as Alzheimer's disease and Lou Gehrig's disease, the hypofunction of a lysosome is the major physiological phenomenon, and the lysosome is a cell organelle that leads circulation and degradation associated with autophagy and endocytosis. The hypofunction of a lysosome commonly occurs in neurodegenerative diseases such as Alzheimer-type dementia, Parkinson's disease, Huntington's disease and Lou Gehrig's disease. Specifically, it has been confirmed that when the function of a lysosome is suppressed, condensation and accumulation of mutant proteins occur, causing problems in maintaining homeostasis of cell organelles. However, until now, as a drug for treating a neurodegenerative disease, there have been attempts to develop an acetylcholinesterase inhibitor or a memantine or N-methyl-D-aspartate channel inhibitor is known, and various drugs such as a secretase inhibitor, but there has not been any research on whether the function or autophagy of lysosomes in the neurodegenerative disease can be an alternative to treatment of a new neurodegenerative disease, and its effective therapeutic method has not been developed yet.

The matters described as the above-described background art are only for improving understanding of the background of the present invention, and should not be taken as acknowledging that they correspond to the prior art already known to those of ordinary skill in the art.

### [Disclosure]

### [Technical Problem]

The inventors made earnest attempts to find a novel compound which prevents or has a therapeutic effect on a neurodegenerative disease among conventional safe therapeutic agents which had gone through clinical testing. As a result, the mechanism related to the association of a PDE inhibitor with a cell organelle or an effective effect of the PDE inhibitor in a neurodegenerative disease was evaluated, and thereby the pharmacological mechanisms and effects of various PDE inhibitors in neurodegenerative disease cells/animal models including Alzheimer-type dementia were confirmed, and thus the present invention was completed.

Accordingly, the present invention is directed to providing a pharmaceutical composition for preventing or treating a neurodegenerative disease, which includes a phosphodiesterase (PDE) inhibitor or a pharmacologically acceptable salt thereof as an active ingredient.

The present invention is also directed to providing a pharmaceutical composition for preventing or treating a neurodegenerative disease, which includes amlexanox (AMX) or a pharmacologically acceptable salt thereof as an active ingredient.

The present invention is also directed to providing a pharmaceutical composition for preventing or treating a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine or a pharmacologically acceptable salt thereof as an active ingredient.

The present invention is also directed to providing a food composition for preventing or improving a neurodegenerative disease, which includes a phosphodiesterase (PDE) inhibitor or a sitologically acceptable salt thereof as an active ingredient.

The present invention is also directed to providing a food composition for preventing or improving a neurodegenerative disease, which includes amlexanox or a sitologically acceptable salt thereof as an active ingredient.

The present invention is also directed to providing a food composition for preventing or improving a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine or a sitologically acceptable salt thereof as an active ingredient.

The present invention is also directed to providing a pharmaceutical composition for preventing or treating cell damage, which includes amlexanox or a pharmacologically acceptable salt thereof as an active ingredient.

The present invention is also directed to providing a use of a phosphodiesterase (PDE) inhibitor or a pharmacologically acceptable salt thereof as an active ingredient to prevent or treat a neurodegenerative disease.

The present invention is also directed to providing a use of amlexanox or a pharmacologically acceptable salt thereof as an active ingredient to prevent or treat a neurodegenerative disease.

The present invention is also directed to providing a use of one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine or a pharmacologically acceptable salt thereof as an active ingredient to prevent or treat a neurodegenerative disease.

The present invention is also directed to providing a method of preventing or treating a neurodegenerative disease by administering a phosphodiesterase (PDE) inhibitor or a pharmacologically acceptable salt thereof into an individual.

The present invention is also directed to providing a method of preventing or treating a neurodegenerative disease by administering amlexanox or a pharmacologically acceptable salt thereof into an individual.

The present invention is also directed to providing a method of preventing or treating a neurodegenerative disease by administering one or more compounds selected from piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine or a pharmacologically acceptable salt thereof into an individual.

Other objects and advantages of the present invention will become more apparent by the following detailed description, claims and drawings.

### [Technical Solution]

Therefore, the present invention provides a pharmaceutical composition for preventing or treating a neurodegenerative disease, which includes a phosphodiesterase (PDE) inhibitor or a pharmacologically acceptable salt thereof as an active ingredient.

The present invention provides a pharmaceutical composition for preventing or treating a neurodegenerative disease, which includes amlexanox or a pharmacologically acceptable salt thereof as an active ingredient.

The present invention provides a pharmaceutical composition for preventing or treating a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient.

The present invention provides food composition for preventing or improving a neurodegenerative disease, which includes a phosphodiesterase (PDE) inhibitor or a sitologically acceptable salt thereof as an active ingredient.

The present invention provides food composition for preventing or improving a neurodegenerative disease, which includes amlexanox or a sitologically acceptable salt thereof as an active ingredient.

The present invention provides a food composition for preventing or improving a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a sitologically acceptable salt thereof as an active ingredient.

The present invention provides a pharmaceutical composition for preventing or treating cell damage, which includes amlexanox or a pharmacologically acceptable salt thereof as an active ingredient.

The present invention provides a use of a phosphodiesterase (PDE) inhibitor or a pharmacologically acceptable salt thereof as an active ingredient to prevent or treat a neurodegenerative disease.

The present invention provides a use of amlexanox or a pharmacologically acceptable salt thereof as an active ingredient to prevent or treat a neurodegenerative disease.

The present invention provides a use of one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient to prevent or treat a neurodegenerative disease.

The present invention provides a method of preventing or treating a neurodegenerative disease by administering a phosphodiesterase (PDE) inhibitor or a pharmacologically acceptable salt thereof into an individual.

The present invention provides a method of preventing or treating a neurodegenerative disease by administering amlexanox or a pharmacologically acceptable salt thereof into an individual.

The present invention provides a method of preventing or treating a neurodegenerative disease by administering one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine or, a pharmacologically acceptable salt thereof into an individual.

In one embodiment of the present invention, the PDE may be one or more selected from the group consisting of PDE1A, PDE1B, PDE1C, PDE2A, PDE3A, PDE3B, PDE4A, PDE4B, PDE5A, PDE6A, PDE6B, PDE6C, PDE6D, PDE7A, PDE7B, PDE8A, PDE8B, PDE9A, PDE10A, and PDE11A, but the present invention is not limited thereto.

In another embodiment of the present invention, the amlexanox or a pharmacologically acceptable salt thereof may prevent or reduce the aggregation of toxic proteins caused by amyloid beta accumulation, but the present invention is not limited thereto.

In still another embodiment of the present invention, the amlexanox or a pharmacologically acceptable salt thereof may prevent or reduce cell death caused by oxidative stress, but the present invention is not limited thereto.

In yet another embodiment of the present invention, the pharmacologically acceptable salt may be an acid-addition salt formed by an organic acid selected from oxalic acid, maleic acid, fumaric acid, malic acid, tartaric acid, citric acid and benzoic acid, or an inorganic acid selected from hydrochloric acid, sulfuric acid, phosphoric acid and hydrobromic acid, but the present invention is not limited thereto.

In yet another embodiment of the present invention, the neurodegenerative disease may be selected from the group consisting of Alzheimer's disease (AD), Parkinson's disease (PD), Huntington's disease, Lou Gehrig's disease (ALS), posttraumatic stress disorder (trauma), multiple sclerosis (MS), cerebral ischemic disease and amyotrophic lateral sclerosis, but the present invention is not limited thereto.

In yet another embodiment of the present invention, the neurodegenerative disease may be Alzheimer's disease.

In yet another embodiment of the present invention, the cell damage may be an increase in lysosome pH by amyloid beta, a decrease in activity of a lysosomal protein or a decrease in zinc concentration in cells, but the present invention is not limited thereto.

In yet another embodiment of the present invention, the cell may be any one or more selected from the group consisting of pericytes, neurons and astrocytes, but the present invention is not limited thereto.

### [Advantageous Effects]

The features and advantages of the present invention are summarized as follows:
i. The present invention provides a pharmaceutical composition for preventing or treating a neurodegenerative disease using a PDE inhibitor whose safety is proven by clinical testing;
ii. The present invention also provides a pharmaceutical composition for preventing or treating amyloid beta-caused cell damage using a PDE inhibitor; and
iii. The composition of the present invention can be effectively used to safely and effectively improve or treat a neurodegenerative disease and cell damage caused by amyloid beta without a side effect.

### [Description of Drawings]

FIG. 1 shows RT-PCR results which confirm mRNA expression of PDE isoforms (1 to 11) in neurons, astrocytes, pericytes and 661W photoreceptor cells (661W).
FIG. 2 shows RT-PCR results that confirm mRNA expression of PDE isoforms (1 to 11) in CHO7WΔE9 cells, SH-SY5Y/swe cells and ARPE-19 cells.
FIG. 3 is a set of time-dependent confocal microscope images of mouse pericytes after being treated with fluorescent dye (FITC)-attached amyloid beta, and a graph of the measurement of the fluorescence intensity of FITC-attached amyloid beta in the cells.
FIG. 4 shows confocal microscope images for analyzing amyloid beta accumulation per cell organelle after mouse pericytes are treated with fluorescent dye (FITC)-attached amyloid beta.
FIG. 5 is a set of confocal microscope images of mouse pericytes which are cultured and then treated with CTL (control), amyloid beta (Aβ) and cilostazol (amyloid beta+cilostazol; +cilo) for 1 hour, respectively.
FIG. 6 shows a western blotting result for mouse pericytes which are cultured and then treated with CTL (control) or amyloid beta (Aβ) and cilostazol (amyloid beta+cilostazol; +cilo) for 24 hours, respectively.
FIG. 7 is a set of images of mouse pericytes, which are cultured, treated with CTL (control), cilostazol (amyloid beta+cilostazol; +cilo) and cAMP (amyloid beta+cAMP) for 1 hour, and then stained with Fluozin-3 (staining zinc in the cytosol) and LysoTracker (staining zinc in acidic cell organelles), respectively.
FIG. 8A is a set of images that visualize pH changes for 0 to 1 hour in respective groups of mouse astrocytes, which are stained with LysoSensor Green DND-189, and then treated with either amyloid beta (Aβ) or amyloid beta and cilostazol (Aβ+cilo).
FIG. 8B is a set of images that visualize pH changes in respective groups of mouse astrocytes, which are stained with an acridine orange dye, and then treated with nothing (CTL; control), bafilomycin A1 (BA), bafilomycin+ cilostazol (+cilo) or bafilomycin + cAMP (+cAMP).
FIG. 9 shows the experimental method and device for HTS.
FIG. 10 is a graph representing luminescence raw values according to cAMP concentrations in the presence of PDE and cAMP
FIG. 11 shows the result of a PDE3 concentration-dependent test, represented by a set of graphs of luminescence raw values according to PDE3 concentrations and percent activity (% activity) according to PDE3 concentrations.
FIG. 12 is a set of graphs representing luminescence raw values for PDE3 according to cilostazol (positive control) concentrations and percent activity (% activity) according to the concentration of cilostazol, which is a PDE3 inhibitor.
FIG. 13 is a graph representing Z' scores for the library of 800 FDA-approved drugs for PDE3.
FIG. 14 is a set of graphs for analyzing percent activity (% activity) according to concentrations of PDE isoforms.
FIG. 15 is a set of graphs for analyzing percent activity (% activity) according to concentrations of primarily-selected compounds (Z001 to Z011) for PDE3.
FIG. 16 is a graph representing the percent activity (% activity) according to the concentrations of finally selected amlexanox (AMX) for various PDE isoforms.
FIGS. 17 and 18 show western blotting images representing the amyloid beta expression pattern and G93A SOD-1 and P301L tau expression when amyloid beta-accumulated pericytes and astrocytes are treated with the finally selected amlexanox (AMX).
FIG. 19 is a set of time-dependent confocal microscope images (left) of mouse pericytes when being treated with Bafilomycin A1, which is a vATPase inhibitor, and then stained with LysoSensor, and quantified data (right) thereof.
FIG. 20 is a set of confocal microscope images of groups of mouse pericytes, which are not treated (CTL) or treated with finally-selected amlexanox (AMX) (PDEi), in which, to measure a zinc concentration, fluorescence intensity was detected by treating the cells with a fluorescent dye (Zinpyr-1) for detecting zinc ions.
FIG. 21 is a set of time-dependent confocal microscope images of mouse astrocytes, which are treated with bafilomycin A1, which is a vATPase inhibitor, to assess the degree of hydrolysis of cathepsin B (top) and cathepsin L (bottom).
FIG. 22 is a set of time-dependent confocal microscope images of bafilomycin A1-treated mouse astrocytes, which are treated with finally selected amlexanox (AMX; Z007), to assess the degree of hydrolysis of cathepsin B (top) and cathepsin L (bottom).
FIG. 23 is a diagram illustrating the test schedule for evaluating the pharmacological efficacy of amlexanox (AMX) in Alzheimer's disease animal models, 5X FAD ((Swedish mutation (KM670/671NL: an increase in Aβ production), Florida mutation (I716V: an increase in Aβ42 production), London mutation (V717I: an increase in Aβ42 production), human presenilin 1 (M146L and L286V: an increase in Aβ42 production) mice).
FIGS. 24 and 25 are a set of graphs representing the pharmacological efficacy of amlexanox (AMX) on a decrease in cognitive memory by amyloid beta (Aβ1-42) and PS1 overexpression, analyzed by water maze evaluation consisting of a hidden platform test and a probe test (top: 1 mg/kg, bottom: 2.5 mg/kg).
FIG. 26 shows the results of experiments performed by dividing Lou Gehrig's disease animal models (G93A SOD-1 mice) into two groups (AMX-treated group and control (Veh)).
FIG. 27 is a graph obtained by inducing cell death by causing oxidative damage to astrocytes with H₂O₂, and treating the cells with amlexanox (AMX) to quantify the cell death using an LDH method.
FIG. 28 is a result confirming the inhibitory effect of amlexanox (AMX) on GFP-mHttQ aggregation.
FIG. 29 is a result confirming the effect of improving a survival rate by amlexanox (AMX) in a G93A-SOD mouse model.
FIG. 30 is a result confirming the ataxia relieving effect of amlexanox (AMX) in relation to the delay of disease progression in a G93A-SOD mouse model.
FIG. 31 is a graph representing the percent activity (% activity) according to piceathanol concentrations for various PDE isoforms.
FIG. 32 is a graph representing the percent activity (% activity) according to ataluren concentrations for various PDE isoforms.
FIG. 33 is a graph representing the percent activity (% activity) according to masitinib concentrations for various PDE isoforms.
FIG. 34 is a graph representing the percent activity (% activity) according to JTC-801 concentrations for various PDE isoforms.
FIG. 35 is a graph representing the percent activity (% activity) according to obatoclax mesylate concentrations for various PDE isoforms.
FIG. 36 is a graph representing the percent activity (% activity) according to dovitinib concentrations for various PDE isoforms.
FIG. 37 is a graph representing the percent activity (% activity) according to CYC116 concentrations for various PDE isoforms.
FIG. 38 is a graph representing the percent activity (% activity) according to resveratrol concentrations for various PDE isoforms.
FIG. 39 is a graph representing the percent activity (% activity) according to pifithrin concentrations for various PDE isoforms.
FIG. 40 is a graph representing the percent activity (% activity) according to 5,5'-(2,5-furandiyl)bis-2-thiophenemethanol (RITA) concentrations for various PDE isoforms.
FIG. 41 is a graph representing the percent activity (% activity) according to axitinib concentrations for various PDE isoforms.

### [Modes of the Invention]

The present invention provides a pharmaceutical composition for preventing or treating a neurodegenerative disease, which includes a phosphodiesterase (PDE) inhibitor or a pharmacologically acceptable salt thereof as an active ingredient.

The present invention provides a pharmaceutical composition for preventing or treating a neurodegenerative disease, which includes amlexanox (AMX) or a pharmacologically acceptable salt thereof as an active ingredient.

The present invention provides a pharmaceutical composition for preventing or treating a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient.

As a result of endeavoring to discover a novel material that inhibits amyloid plaque and tau protein expression in neurons, astrocytes and pericytes, caused by amyloid beta accumulation in various cells of mice, and mitigates or restores the decrease in activity of organelles in cells, the inventors confirmed that amlexanox used as a PDE inhibitor is able to effectively treat a neurodegenerative disease such as Alzheimer's disease or Lou Gehrig's disease, occurring due to amyloid beta accumulation.

In the specification, the term "phosphodiesterase (PDE) inhibitor" (hereinafter, a PDE inhibitor) may be, for example, amlexanox, piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, or silver sulfadiazine, but the present invention is not limited thereto.

In the specification, the term "neurodegenerative disease" refers to abnormality in motor control ability, cognitive function, perceptive function, sensory function and autonomic function due to toxic proteins generated by induction of abnormal aggregation of amyloid beta and abnormal changes in tau protein through several cells or a signaling system by changes triggered by the accumulation of amyloid beta, and has the same meaning as a "degenerative brain disease." The neurodegenerative disease may be classified by clinical characteristics. Examples of diseases exhibiting progressive cognitive impairment as a main symptom include Alzheimer's disease, dementia (frontotemporal dementia, dementia with Lewy bodies, etc.) and corticobasal degeneration, and examples of diseases exhibiting progressive ataxia include Parkinson's disease, multiple system atrophy, Huntington's disease and progressive supranuclear palsy. Examples of neurodegenerative diseases exhibiting symptoms of hypotonia and myoatrophy include Lou Gehrig's disease, primary lateral sclerosis, and spinal muscular atrophy.

The term "amyloid plaque" used herein may be an insoluble fibrous protein aggregate including amyloid beta. The amyloid plaque may be present in a cell, on the surface of a cell, and/or in the space between cells. For example, the amyloid plaque may be present in the space between cells of nervous tissue.

The term "nervous tissue" used herein includes central nervous system tissue such as the brain. Brain tissue may include the cerebrum, the cerebellum and hippocampal tissue. The cerebral tissue includes the cerebral cortex. Nervous tissue is the nervous tissue itself as well as including a neuron, and the neuron is one of the components constituting the nervous tissue. The neuron includes neuronal cells and/or astrocytes. The culture of nervous tissue includes the culture of nervous cells such as neuronal cells and/or astrocytes in vitro.

The term "treatment" used herein refers to all actions involved in alleviating or beneficially changing symptoms caused by the administration of a composition according to the present invention. Those of ordinary skill in the technical field to which the present invention belongs will identify the exact criteria of a disease in which the composition of the present invention is effective and determine the degree of alleviation, improvement and treatment with reference to the data presented by the Korean Medical Association, etc.

The term "prevention" used herein refers to all actions of inhibiting or delaying the onset of a related disease by the administration of the composition according to the present invention. For example, by a neuropsychological test, it is possible to diagnose pre-dementia patients with mild cognitive impairment, average 12% of patients with mild cognitive impairment for 1 hour develop Alzheimer's disease, and when the mild cognitive impairment goes untreated for approximately 6 years, 80% of patients develop Alzheimer's disease, and therefore when the composition of the present invention alleviates or reduces toxicity caused by toxic proteins such as an amyloid plaque including amyloid beta and an abnormal tau protein is administered, an effect of preventing or delaying the progression of a neurodegenerative disease may be exhibited.

According to the embodiment of the present invention, the composition of the present invention prevents or reduce the aggregation of toxic proteins caused by the accumulation of amyloid beta, in drug administration and behavior evaluation experiments with an Alzheimer-type dementia-induced animal model, that is, 5X FAD mice, the composition of the present invention exhibited efficacy in preventing the loss of or restoring memory and learning ability (Example 12), and in drug administration and survival rate evaluation tests with G93A SOD-1 mice in which a mutant protein of the ALS-related gene, SOD-1 (the expression of an amino acid at codon 93, in which glycine is substituted with alanine), of a human, which is a Lou Gehrig's disease animal model, is expressed, the time of the onset of Lou Gehrig's disease and the efficacy of prolonging a survival rate were confirmed (Example 13). In addition, in an in vitro experiment with oxidative cell damage-induced astrocytes, cell death was significantly reduced (Example 14).

Amlexanox has an IUPAC name of 2-amino-7-isopropyl-5-oxo-5H-[1]benzopyrano[2,3-b] pyridine-3-carboxylic acid, a molecular formula of C₁₆H₁₄N₂O₄, and a molecular weight of 298.30. Amlexanox is odorless and is an off-white crystalline powder, and its structural formula is as follows:

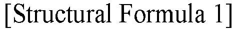

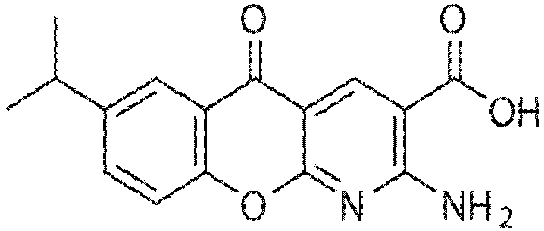

Amlexanox is a compound which is used as an anti-allergic drug with a clinically proven effect on atopic diseases, particularly, allergic asthma and rhinitis and having secured safety.

While there are various compounds having similar structural formulas to amlexanox, among these compounds, it was confirmed that one that exhibits an equivalent or better effect even when a significantly lower concentration, which is 7 to 40-fold lower than the others, is used and an excellent reducing efficacy in all PDE isoforms is amlexanox (AMX; Z007) (Examples 8 and 9).

The PDE inhibitor of the present invention may be one that is listed in Table 1 below, but the present invention is not limited thereto:

**[Table 1]**

| **Name** | **Structural formula** | **Molecular weight (g/mol)** |
|---|---|---|
| Piceathanol | 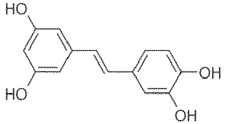 | 244.24 |
| Ataluren | 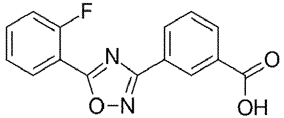 | 284.24 |
| Masitinib | 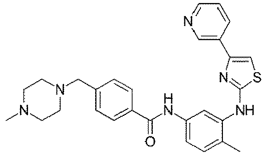 | 498.64 |
| JTC-801 | 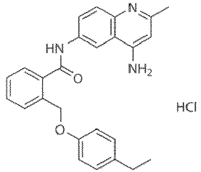 | 411.51 |
| Obatoclax mesylate | 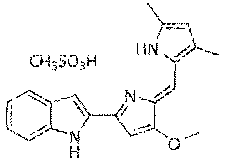 | 413.49 |
| Dovitinib | 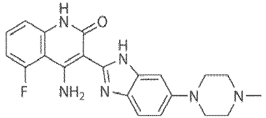 | 392.43 |
| CYC116 | 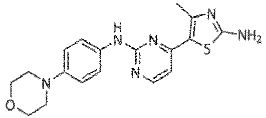 | 368.46 |
| Resveratrol | 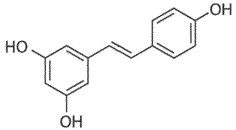 | 228.24 |
| Pifithrin | 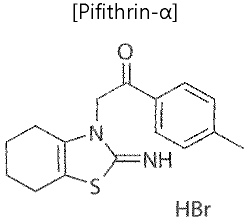 | 367.30 |
| | 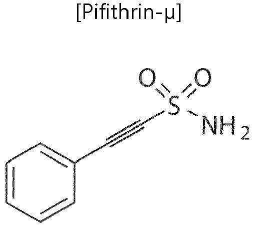 | 181.21 |
| RITA(5,5'-(2,5-furandiyl)bis-2-thiophene methanol) | 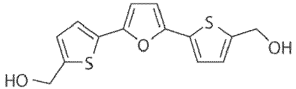 | 292.37 |
| Axitinib | 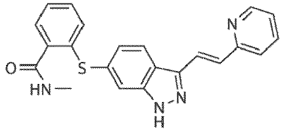 | 386.47 |
| Imatinib mesylate | 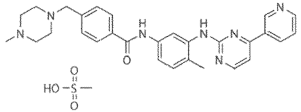 | 589.71 |
| Zafirlukast | 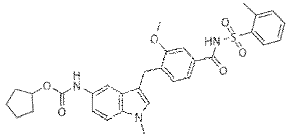 | 575.68 |
| Hexachloropene | 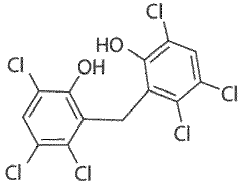 | 406.9 |
| Febuxostat | 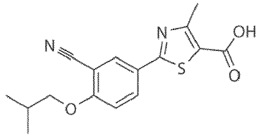 | 316.37 |
| Silver Sulfadiazine | 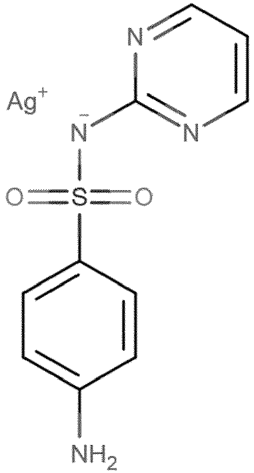 | 357.14 |

The expression that something is "included as an active ingredient" used herein refers that something is included at a sufficient amount for achieving the efficacy or activity of the PDE inhibitor or amlexanox. The PDE inhibitor or amlexanox in the composition of the present invention may be included at, for example, 0.001 mg/kg or more, preferably 0.1 mg/kg or more, more preferably 1 mg/kg or more, and still more preferably 10 mg/kg or more. Even when an excessive amount of the PDE inhibitor or amlexanox is administered, there are almost no side effects in the human body, the upper limit of the quantity of the PDE inhibitor or amlexanox included in the composition of the present invention may be selected by one of ordinary skill in the art within a suitable range.

The PDE inhibitor or amlexanox that is used as an active ingredient in the composition of the present invention is interpreted as a meaning of the compound itself, as well as including a pharmacologically or sitologically acceptable salt, hydrate, solvate or prodrug thereof.

According to an exemplary embodiment of the present invention, the composition of the present invention is a pharmaceutical composition.

The term "pharmacologically acceptable salt" used herein refers to a dosage form of a compound that does not induce severe irritation in an organism to which the compound is administered and damage the biological activity and physical properties of the compound.

In the pharmacologically acceptable salt, as a pharmacologically acceptable acid, for example, an organic acid such as oxalic acid, maleic acid, fumaric acid, malic acid, tartaric acid, citric acid or benzoic acid, or an inorganic acid such as hydrochloric acid, sulfuric acid, phosphoric acid or hydrobromic acid is included.

In addition, the pharmacologically acceptable salt may be obtained by reacting the compound of the present invention with a base, thereby forming an ammonium salt, an alkali metal salt such as a sodium or potassium salt, an alkaline earth metal salt such as a calcium or magnesium salt, a salt of an organic base such as dicyclohexylamine, N-methyl-D-glucamine or tris(hydroxymethyl) methylamine, or a salt of an amino acid such as arginine or lysine, but the present invention is not limited thereto.

The term "pharmacologically acceptable hydrate" used herein refers to a hydrate of the PDE inhibitor or amlexanox having a desired pharmacological effect. The term "pharmacologically acceptable solvate" refers to a solvate of a compound of the PDE inhibitor or amlexanox having a desired pharmacological effect. The hydrate and solvate may also be prepared using the above-described acid.

The term "pharmacologically acceptable prodrug" refers to a derivative of the PDE inhibitor or amlexanox that has to undergo bioconversion before exerting the pharmacological effect of the inhibitor or amlexanox. To improve chemical safety, patient acceptance, bioavailability, organ selectivity or ease of preparation, such a prodrug is prepared to reduce the prolonging of the duration of action and side effects. The product of the present invention may be easily prepared using amlexanox according to the method conventionally used in the art (e.g., Burger's Medicinal Chemistry and Drug Chemistry, 5th ed., 1:172-178 and 949-982(1995)).

A pharmaceutically acceptable carrier included in the pharmaceutical composition of the present invention is conventionally used in formulation, and includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil, but the present invention is not limited thereto. The pharmaceutical composition of the present invention may further include a lubricant, a wetting agent, a sweetening agent, a flavoring agent, an emulsifier, a suspending agent and a preservative, other than the above-described components. Suitable pharmaceutically acceptable carriers and formulations are described in detail in the Remington's Pharmaceutical Sciences (19th ed., 1995).

The pharmaceutical composition of the present invention may be administered orally or parenterally, and parenteral administration may include intranasal administration, intraocular administration, intravenous injection, subcutaneous injection, intramuscular injection, intraperitoneal injection, or transdermal administration. A suitable dosage of the pharmaceutical composition of the present invention varies depending on factors such as a formulation method, the mode of administration, the age, body weight, sex and pathological condition of a patient, food, administration time, the route of administration, an excretion rate and reaction sensitivity, and normally skilled physicians are able to be easily determine and prescribe a dosage effective in desired treatment or prevention. According to an exemplary embodiment of the present invention, a daily dose of the pharmaceutical composition of the present invention is 0.001 to 100 mg/kg.

The pharmaceutical composition of the present invention may be formulated using a pharmaceutically acceptable carrier and/or an excipient, and thus prepared in a unit dose or multidose form. Here, the formulation may be a solution, suspension or emulsion in oil or an aqueous medium, an extract, a powder, a granule, a tablet or a capsule, and may further include a dispersing agent or a stabilizer.

The pharmaceutical composition of the present invention may be prepared in the formulation of a dermal preparation for external use, an aerosol, a spray, an eye drop, an oral preparation or an injection. The pharmaceutical composition of the present invention may be used for a human or animal.

The present invention also provides a food composition for preventing or improving a neurodegenerative disease, which includes a phosphodiesterase (PDE) inhibitor or a sitologically acceptable salt thereof as an active ingredient.

The present invention also provides a food composition for preventing or improving a neurodegenerative disease, which includes amlexanox or a sitologically acceptable salt thereof as an active ingredient.

The present invention also provides a food composition for preventing or improving a neurodegenerative disease, which includes one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a sitologically acceptable salt thereof as an active ingredient.

The food composition according to the present invention may be used as a functional food or added to various types of food. Examples of the food to which the composition of the present invention may be added include, for example, beverages, alcoholic beverages, confectionery, diet bars, dairy products, meat, chocolate, pizza, bread, ramen, other noodles, gums, ice cream, vitamin complexes, and health supplements.

The food composition of the present invention may include a PDE inhibitor or amlexanox as an active ingredient as well as conventionally added components in food production, which include, for example, a protein, a carbohydrate, a lipid, a nutrient, a seasoning and a flavoring agent. Examples of the above-mentioned carbohydrates may include common sugars, for example, monosaccharides such as glucose and fructose; disaccharides such as maltose, sucrose and oligosaccharides; and polysaccharides such as dextrin and cyclodextrin, and sugar alcohols such as xylitol, sorbitol and erythritol. As a flavoring agent, a natural flavoring agent [taumatin, stevia extract (e.g., rebaudioside A, glycyrrhizin, etc.)] and a synthetic flavoring agent (saccharin, aspartame, etc.) may be used. For example, when the food composition of the present invention is produced in drinks and beverages, other than amlexanox, citric acid, liquid fructose, sugar, glucose, acetic acid, malic acid, fruit juice and various plant extracts may be additionally included.

The present invention provides a health functional food as a food composition including a PDE inhibitor, amlexanox or a sitologically acceptable salt thereof as an active ingredient. Health functional foods are foods prepared by adding amlexanox to food ingredients for beverages, tea, spices, chewing gum and confectioneries, or prepared in a capsule, powder or a suspension, and when ingested, it means that they have specific effects on health, but unlike general drugs, they use food as a raw material and thus have no side effects that occur in long-term administration of a drug. The health functional food of the present invention obtained thereby can be ingested daily, and thus is very useful. In the health functional food, an amount of the added PDE inhibitor or amlexanox may not be uniformly regulated depending on a type of the corresponding health functional food, but added in a range without damaging the original taste of the food, and the range is generally 0.01 to 50 wt%, preferably 0.1 to 20 wt% with respect to the corresponding food. In addition, in the case of the health functional food in a pill, granule, tablet or capsule form, the PDE inhibitor or amlexanox may be usually included at 0.1 to 100 wt%, and preferably, 0.5 to 80 wt%. In one embodiment, the health functional food of the present invention may be formulated in a pill, tablet, capsule or beverage.

The term "sitologically acceptable salt" used herein refers to the formulation of a compound that does not induce serious irritation in an organism to which a compound is administered and damage the biological activity and physical properties of the compound. The sitological salt may be obtained by reacting the compound of the present invention with an inorganic acid such as hydrochloric acid, bromic acid, sulfuric acid, nitric acid or phosphoric acid, a sulfonic acid such as methanesulfonic acid, ethanesulfonic acid or p-toluenesulfonic acid, or an organic carbonic acid such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid or salicylic acid. In addition, the sitological salt may be obtained by forming an ammonium salt, an alkali metal salt such as a sodium or potassium salt, an alkaline earth metal salt such as a calcium or magnesium salt, a salt of an organic base such as dicyclohexyl amine, N-methyl-D-glucamine or tri(hydroxymethyl)methylamine, or a salt of an amino acid such as arginine or lysine through a reaction between the compound of the present invention and a base, but the present invention is not limited thereto.

The food composition of the present invention may be used as human food, or animal feed or feed additive.

According to yet another aspect of the present invention, the present invention provides a pharmaceutical composition for preventing or treating cell damage, which includes amlexanox or a pharmacologically acceptable salt thereof as an active ingredient.

As a result of endeavoring to discover a novel material that has protective activity against cell damage caused by toxic proteins such as an amyloid plaque and a tau protein in neurons, astrocytes and pericytes, which are shown by the accumulation of amyloid beta in various mouse cells and less toxicity and side effects even in long-term administration, the inventors confirmed that amlexanox newly found as a PDE inhibitor can effectively treat cell damage such as a pH change in lysosomes, degradation in activity of a lysosomal protein or a decrease of an intracellular zinc concentration in cells occurring due to the accumulation of amyloid beta.

The term "amyloid beta-caused cell damage" used herein refers to cell damage in the body, which is caused by toxic proteins generated by induction of abnormal aggregation of amyloid beta and abnormal changes in tau protein through several cells or a signaling system by changes triggered by the accumulation of amyloid beta. Specifically, the amyloid beta-caused cell damage means that the lysosome pH may increase, the activity of a lysosomal protein may be degraded, a zinc concentration in cells may decrease, or cell death may be induced. The amyloid beta-caused cell damage may be caused by a neurodegenerative disease, a degenerative brain disease, a spinal cord injury or peripheral nerve damage, but the present invention is not limited thereto. The neurodegenerative disease may be classified by clinical characteristics, and examples of diseases exhibiting progressive cognitive impairment as a main symptom include Alzheimer's disease, dementia (frontotemporal dementia, dementia with Lewy bodies, etc.) and corticobasal degeneration, and examples of diseases exhibiting progressive ataxia include Parkinson's disease, multiple system atrophy, Huntington's disease and progressive supranuclear palsy. Examples of neurodegenerative diseases exhibiting symptoms of hypotonia and myoatrophy include Lou Gehrig's disease, primary lateral sclerosis, and spinal muscular atrophy.

The term "treatment" used herein refers to all actions involved in alleviating or beneficially changing symptoms caused by the administration of a composition according to the present invention. Those of ordinary skill in the technical field to which the present invention belongs will identify the exact criteria of a disease in which the composition of the present invention is effective and determine the degree of alleviation, improvement and treatment with reference to the data presented by the Korean Medical Association, etc.

The term "prevention" used herein refers to all actions of inhibiting or delaying the onset of a related disease by the administration of the composition according to the present invention. For example, by a neuropsychological test, it is possible to diagnose pre-dementia patients with mild cognitive impairment, average 12% of patients with mild cognitive impairment for 1 hour develop Alzheimer's disease, and when the mild cognitive impairment goes untreated for approximately 6 years, 80% of patients develop Alzheimer's disease, and therefore when the composition of the present invention alleviates or reduces toxicity caused by toxic proteins such as an amyloid plaque including amyloid beta and an abnormal tau protein is administered, an effect of preventing or delaying the progression of a neurodegenerative disease may be exhibited.

According to an embodiment of the present invention, it was confirmed that, when amyloid beta was injected and accumulated in pericytes, and then AMX was treated, the aggregation of amyloid beta and a tau protein was inhibited. In addition, in addition to the accumulation of amyloid beta, it was confirmed that the accumulation of toxic proteins such as Tau and G93A SOD-1 may also be reduced by AMX (Example 9).

In addition, it was confirmed that when amlexanox (AMX) was administered into astrocytes in which lysosome pH in the cells were alkalified by administration of bafilomycin A1, a lysosome pH environment was restored (acidified) to the original state within 60 minutes, and the concentration of zinc in the cells was restored and increased to a normal level (Example 10).

In addition, when astrocytes in which lysosomal protein activity is inhibited by bafilomycin A1 are treated with amlexanox, it can be confirmed that the astrocyte's lysosome activity was restored to the original state within 60 minutes (Example 11).

In addition, according to an embodiment of the present invention, the protective effect of the composition of the present invention against damage of astrocytes receiving oxidative stress (glutamate or hydrogen peroxide treatment) caused by toxic proteins generated by amyloid beta accumulation was confirmed using an LDH method. As a result, it was confirmed that the survival rate in a cell group treated with the composition of the present invention was significantly high (40%) (Example 14), and there was almost no morphological change caused by cell damage.

The pharmaceutical composition according to the present invention for preventing or treating amyloid beta-caused cell damage, which includes amlexanox as an active ingredient, may be used as a medicine having a therapeutic or preventive effect against cell damage.

The amlexanox is a compound with secured safety since it is use as an antiallergic drug clinically proven to be effective in atopic diseases, particularly, allergic asthma and rhinitis.

The present invention provides a use of a phosphodiesterase (PDE) inhibitor or a pharmacologically acceptable salt thereof as an active ingredient to prevent or treat a neurodegenerative disease.

The present invention provides a use of amlexanox or a pharmacologically acceptable salt thereof as an active ingredient to prevent or treat a neurodegenerative disease.

The present invention provides a use of one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient to prevent or treat a neurodegenerative disease.

The present invention provides a method of preventing or treating a neurodegenerative disease by administering a phosphodiesterase (PDE) inhibitor or a pharmacologically acceptable salt thereof into an individual.

The present invention provides a method of preventing or treating a neurodegenerative disease by administering amlexanox or a pharmacologically acceptable salt thereof into an individual.

The present invention provides a method of preventing or treating a neurodegenerative disease by administering one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof into an individual.

The term "administration" used herein refers to introduction of the pharmaceutical composition of the present invention to a patient by any suitable method, and the administration route of the composition of the present invention may be administered through various oral or parenteral routes as long as reaching target tissue.

The term "individual" used herein refers to a subject to which a PDE inhibitor, a pharmaceutically acceptable salt thereof, or a pharmaceutical composition including the same can be administered, but there is no limitation to the subject. The individual of the present invention is preferably a mammal including a human, for example, a mammal such as a human, a mouse, a rate, a monkey, a cat, a dog, a cow or a pig, but the present invention is not limited thereto.

Hereinafter, the present invention will be described in further detail with reference to examples. These examples are merely provided to illustrate the present invention, and it should not be construed by those of ordinary skill in the art that the scope of the present invention is limited by the following examples according to the gist of the present invention.

### <Experimental methods and materials >

Reagents and protein dyes used herein were purchased or prepared as follows: Amlexanox, bafilomycin A1 and hydrogen peroxide were purchased from Sigma, beta amyloid (6E10) was purchased from Biolegend, and a green fluorescent protein (GFP) was purchased from Santa Cruz. In addition, GM130 antibodies were purchased from Epitomics, EEA1 antibodies were purchased from Cell Signaling, and LAMP1 antibodies were purchased from Novus Biological.

As dyes for detecting zinc activity in cells, FluoZin-3 was purchased from Molecular Probe, Zinpyr-1 was purchased from Mellitech, and Lysosensor green DND-189 was purchased from Molecular Probe.

In addition, beta-amyloid 1-42, which is a sample used to accumulate amyloid beta in cells, was purchased from American Peptide Company.

### 1. Drug screening

For drug screening, high-throughput screening (HTS) was used. Specifically, an effective material was acquired using a conventional PDE inhibitor and searching an FDA-approved drug library and the Selleckchem library based on cAMP.

### 2. Cell culture

### 1) Cortical astrocyte culture

For astrocyte culture, first, cerebral cortices (neocortices) were dissociated from murine fetuses (3 weeks old), and the obtained cortical tissue was divided into single cells. Subsequently, the single cells were seeded, and the cultured astrocytes were stored using a culture medium (DMEM 7% FBS + 7% HS + 100 U/mL penicillin + 100 µg/mL glutamine) in a humidified incubator at 37 °C under a condition of 5% carbon dioxide. After DIV 10-14, cells grown to 80-90% or more confluence were used, and before an experiment, the cells were washed twice with warm MEM and used. New cells were cultured and used each time without passaging.

### 2) Pericyte culture

Cerebral cortices (neocortices) of 3 to 4-week-old mice were dissociated, and the separated tissue was divided into single cells using collagenase. Afterward, a blood vessel cell layer was separated using Percoll to isolate cells. The cells were stored for approximately 2 weeks using a culture medium (DMEM + 20% FBS + 100 U/mL penicillin + 100 µg/mL glutamine) in a humidified incubator at 37 °C under a condition of 5% carbon dioxide. After culture, the cells were subcultured to obtain cells at passage 4 or more, and the resulting cells were used for an experiment. Before the experiment, the cells were washed twice with warm MEM and used.

### 3) 661W photoreceptor cells (661W)

Cells provided by the University of Oklahoma were cultured in a plate coated with fibronectin or poly-L-Lysine (PLL). The culture cells were stored in a culture medium (DMEM + 10% FBS + 100 U/mL penicillin) in a humidified incubator at 37 °C under a condition of 5% carbon dioxide. Whenever needed for the experiment, the cells were trypsinized and subcultured until full confluence (100% confluent culture).

### 4) Human retinal pigment epithelial cells (ARPE-19)

Cells were purchased from a cell line bank (ATCC CRL-2302, the American Type Culture Collection, Manassas, VA, USA). The cultured cells were stored in a culture medium (DMEM + 5% FBS + 5% HS + 100 U/mL penicillin + 200 µM glutamine) in a humidified incubator at 37 °C under a condition of 5% carbon dioxide. Whenever needed for the experiment, the cells were trypsinized and subcultured until full confluence (100% confluent culture).

### 5) CHO7WΔE9 cells

The Chinese hamster ovary 7WΔE9(CHO7WΔE9) cell line was provided by Dr. David E. Kang (USF Health Byrd Alzheimer's Institute, Tampa, FL, USA). The cultured cells were stored in a culture medium (RPMI1640 + 10% FBS + 5% HS + 100 U/mL penicillin) in a humidified incubator at 37 °C under a condition of 5% carbon dioxide. Whenever needed for the experiment, the cells were trypsinized and subcultured until full confluence (100% confluent culture).

### 6) SH-SY5Y cells

SH-SY5Y cells were stored using a Dulbecco's modified Eagles medium (DMEM; Gibco) containing 10% (v/v) fetal bovine serum (FBS), 100 U/mL penicillin/streptomycin in a humidified incubator at 37 °C under a condition of 5% carbon dioxide. Whenever needed for the experiment, the cells were trypsinized and subcultured until full confluence (100% confluent culture).

### 3. Confocal microscopy

Fluorescent dyes such as LysoSensor (measurement of pH in cells; green) and LysoTracker (staining cell organelles; red) were treated for 30 minutes using a live-cell confocal microscope, the medium was exchanged with a medium containing a live-cell imaging solution, and then the structures of cell organelles and pH changes were observed in real time using a live-cell confocal microscope.

### 4. Immunostaining

The pericytes cultured under the above-described culture conditions were fixed with 4% paraformaldehyde for 30 minutes, washed with PBS three times or more, blocked with a blocking solution (1% BSA+ 0.1% Triton X-100) for 30 minutes, and reacted with primary antibodies overnight. The primary antibodies used herein include a Golgi matrix marker (GM130), an endosome marker (EEA1) and a lysosome marker (LAMP1). After the primary antibodies were stained, the cells were washed with PBS for three times or more and reacted with secondary antibodies (Alexa Fluor-conjugated secondary antibodies) for 1 hour, followed by making a specimen on a glass plate.

### 5. Western blot analysis

Each type of cell was cultured under the culture conditions, a cell culture was restored, followed by preparing a particulate fraction in a 50 mM Tris buffer. Proteins obtained from the cell culture developed in a SDS polyacrylamide gel, transferred to an immobilon-P membrane (Millipore, USA), and reacted with antibodies overnight. Next day, western blots were washed, reacted with peroxidase-linked secondary antibodies at room temperature for 1 hour, followed by staining bands with 4-chloro-1-naphthol.

### 6. Reverse transcription-polymerase chain reaction (RT-PCR)

RNA was isolated from the cells using a TRIsure solution (BIOLINE, BIO-38032), and purified using isopropanol and ethanol. cDNA was synthesized from RNA through reverse transcription using QuantiTect Reverse Transcription Kit (Qiagen, Cat No./ID: 205311). An expression level of PDE mRNA in cells was determined using the cDNA through polymerase chain reaction (PCR Master Mix; Thermo Scientific, #K0171).

Primer sequences of PDE isoforms used in PCR are as follows:
PDE1A-F_GAAGCAGCTGGAGAAAGGTGATGTC
PDE1A-R_CCTTTCCACAAAAATCCCAGCCTGG
PDE1B-F_GCGCTACATGGTGAAGCAGTTGGAG
PDE1B-R_CCACGAAGATCCCAGCCTGCAC
PDE1C-F CGGCTGCCATCCATGACTATGAAC
PDE1C-R GTTCGAAACTCCCTCCAGTCATCC
PDE2A-F GGT CAT ACC CCT GCT GGA CAA GG
PDE2A-R CAGTTGCAGGATCTTTCGGTCATGG
PDE3AF_ACTTTCATGCTTTGGAGATCGGATA
PDE3AR_AGGAATCGGCTGTGTTGTGAGA
PDE3B-F CTCTGGGACTGGGACTTGAAGCAG
PDE3B-R CTTTGGCCTACAGGAACCTGAGAAAG
PDE4A-F GCTTGGAGGCAGAAAATGGGCC
PDE4A-R CGGAGACTGGCCAGCACCTG
PDE4B-F_GTGGAAAATGGCCCTTCTCCAGGTC
PDE4B-R_GGCAAAAGGAGTGACAATCAGGTCATC
PDE5A-F_CGCCTCTTTGATGTTGCTGAAGGTTC
PDE5A-R_CCTGATTTCTTGTTGATGGCCTGAGC
PDE6A-F_GATGTGTGGCCAGTTCTGATGGGC
PDE6A-R_CATCCAGAGGCTCTTTCTGGAATTC
PDE6B-F_GATAAACGTGCAGGATGTGGCAGAG
PDE6B-R_CACAGTTGTGGAGGTAGCTTAGGTG
PDE6C-F_CCGGAGAAGCCAGATCCTTATGTGG
PDE6C-R CCTGCCATCTGGAGTCTTTGGTCC
PDE6D-F GATGAACCTTCGGGATGCCGAAACAG
PDE6D-R GCCAGGTGTTTGTGGAGTTAGGGATC
PDE7A-F CAGCTCTCGCAGAGACGTGGAG
PDE7A-R CCAGCATACACTTGGCTTGTCCATTG
PDE7B-F GTTGTGTCACCTCTTCAACTCCCATGG
PDE7B-R GCATGATGTCCAGAGGTGTGAGG
PDE8A-F GTCACCAAGGAGGTGCAGACTGTC
PDE8A-R GAGTTACAAGCCCTGAGTTTCAGCTG
PDE8B-F CAGTGTTTACAGCGTTAGACCACTGTC
PDE8B-R CGAATGAGGCTCTGTCTGAGAGTTGTC
PDE9A-F_GACACCTCGACGTGATGTCCCC
PDE9A-R_CATCTGTGTCACACAGAAGCAGTGC
PDE10A-F_GGGATTGCTGGTCAAGTGGCAAGAAC
PDE10A-R_GCTTCTCCATGGTAACCCTGTAGATGC
PDE11A-F_GCTGGAGGTGGTCAATGACCTC
PDE11A-R_CTGTCGAAGCAACCAGCTCAGCG
GAPDH-F GTCGTGGAGTCTACTGGTGTCTTCAC
GAPDH-R GTTGTCATATTTCTCGTGGTTCACACCC
hPDE1a-F GCTTGTTCTAGCTGCTGAAACTGACTG
hPDE1a-R CTGCTTCCAGCACAGATGCCGC
hPDE1b-F GAGCTTGGACTGGGAGCCCAAAG
hPDE1b-R GCTGACTTCAGCTCCAGGGGTG
hPDE1c-F GACAGCCCAAACTATCTCAGCTTCAC
hPDE1c-R GATCTACCACTGAAGCTTCCCCTCTC
hPDE3a-F GACCACAAACGAGGGCCAAGAGG
hPDE3a-R CACTGAGCGAAGTGACGGGATTCAC
hPDE3b-F CTGCAGCCAGTTACTATGGCAGTTGC
hPDE3b-R GGC TTG GAT CAG TCA GAA GAT CTG AC
hPDE4a-F CAG GCG CTT CGA GGC AGA GAA TG
hPDE4a-R CCT CGC TGG TGA CCG ATG AGT TC
hPDE4b-F GCC TTT GAC AAC GCT TCC AAG CAT TGC
hPDE4b-R GCT CGC TTG GAA GAG AAG AGT TTC TCG
hPDE5a-F CCAGCAGCAGAAGCAGCAGCAG
hPDE5a-R CAGAGGCAGAGATTTTCCTGGTTGGTG
hPDE7a-F GGAAGTGTGTTACCAGCTGCCGG
hPDE7a-R GGCTCCTTACACGTACATCTCCTAGC
hPDE7b-F CACCGCTCAGAGATTTCACGGCATTC
hPDE7b-R CTTGGTGCCAATCTCCCCTGAGTATG
hPDE10a-F GAGTGCTTCCTGAGCCCCAGTTTG
hPDE10a-R GCTGGTTGTCTCCTCCTGTGTCC
hGAPDH-F GGCACCGTCAAGGCTGAGAACG
hGAPDH-R GCAGAGGGGGCAGAGATGATGAC

### 7. Analysis of lysosome activity

Samples stained with enzymatic dyes such as cathepsin B and cathepsin L were administered into the cell culture to allow a reaction for 30 minutes, and changes in activity of lysosomes in cells were confirmed using a confocal microscope.

### 8. Statistical analysis

Data indicating statistical significance as means ± SEM were measured through deviation analysis using a Scheffe test. A value of P<0.05 was considered statistically significant.

### Experiments and experimental results

### <Example 1> mRNA expression pattern of PDE isoforms by cell type

The central nervous system consists of various cells including neurons and astrocytes. Except neurons and astrocytes, among cells recently attracting attention in terms of neurodegenerative diseases, there are pericytes that regulate the microcirculation of the nervous system. Therefore, this example is for analyzing mRNA expression of PDE isoforms in various cells.

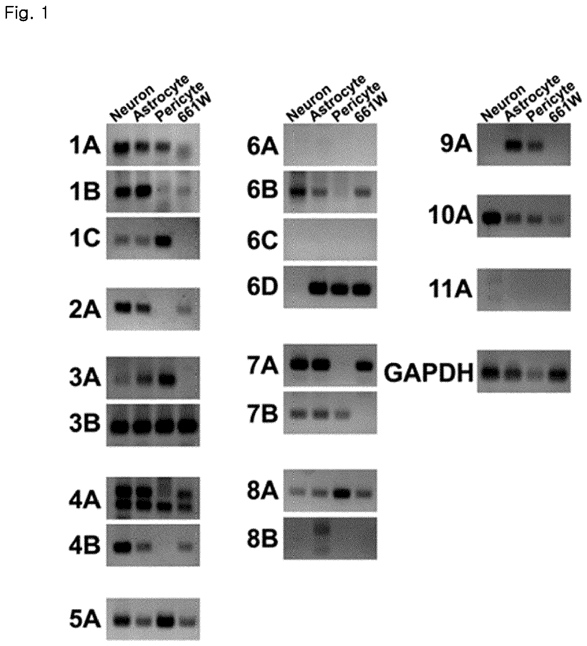

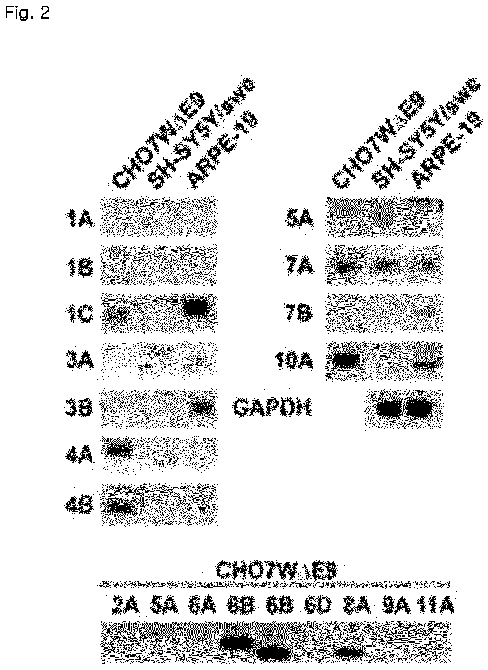

FIG. 1 shows RT-PCR results which confirm mRNA expression of PDE isoforms (1 to 11) in neurons, astrocytes, pericytes and 661W photoreceptor cells (661W). FIG. 2 shows RT-PCR results that confirm mRNA expression of PDE isoforms (1 to 11) in CHO7WΔE9 cells, SH-SY5Y/swe cells and ARPE-19 cells.

As shown in FIGS. 1 and 2, it was confirmed that the PDE isoforms (1 to 11) are expressed in the various cells, and particularly, in neurons, mRNA of PDE 1A, 1B, 1C, 2A, 3A, 3B, 4A, 4B, 5A, 6B, 7A, 7B, 8Aand 10A was expressed, and in astrocytes, an mRNA expression pattern was similar to that in neurons, but the mRNA of the PDE isoforms (1 to 3) was further expressed, compared with neurons. In pericytes, it was able to be confirmed that, although the mRNA expression pattern is similar to that of neurons, mRNA of the PDE isoforms 1 and 2 is less expressed, and mRNA of the PDE isoform 3 is more expressed.

### <Example 2> Analysis of amyloid beta aggregation in pericytes

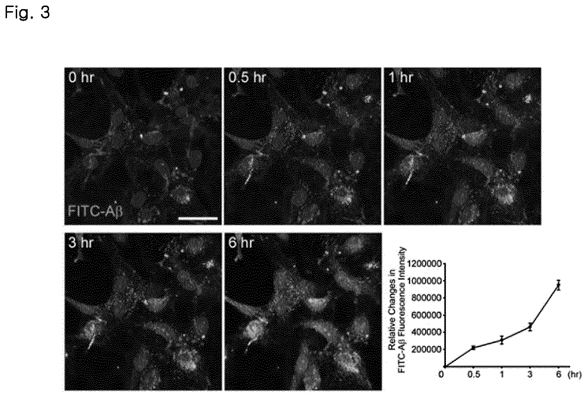

FIG. 3 is a set of time-dependent confocal microscope images of mouse pericytes after being treated with fluorescent dye (FITC)-attached amyloid beta, and a graph of the measurement of the fluorescence intensity of FITC-attached amyloid beta in the cells.

As shown in FIG. 3, when mouse pericytes are isolated and cultured and treated with fluorescent dye-attached amyloid beta, it was confirmed that amyloid beta is introduced into pericytes over time and aggregated into toxic proteins.

### <Example 3> Analysis of amyloid beta aggregation pattern in pericytes

Cell organelles in which amyloid beta is accumulated when amyloid beta is administered into pericytes were localized. To this end, the locations of the cell organelles were detected using antibodies against GM130 (Golgi matrix marker), EEA1 (endosome marker) and LAMP1 (lysosome marker) by a confocal microscope.

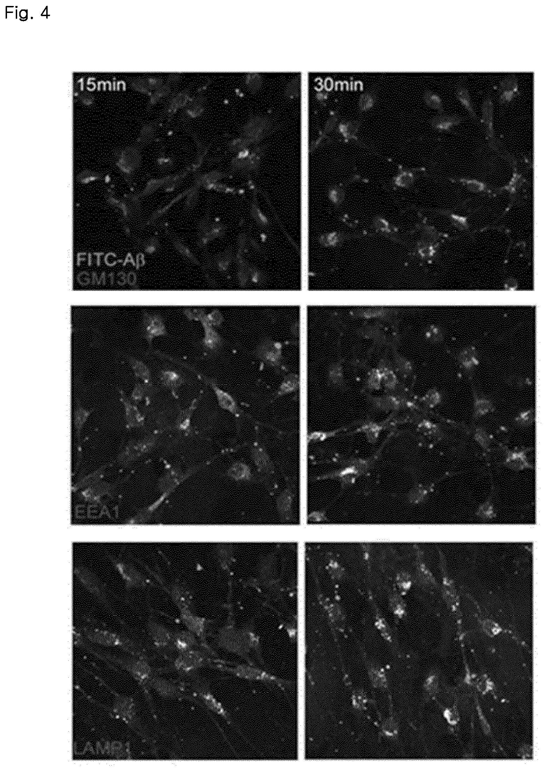

FIG. 4 shows confocal microscope images for analyzing amyloid beta accumulation per cell organelle after mouse pericytes are treated with fluorescent dye (FITC)-attached amyloid beta, confirming that, in pericytes, amyloid beta aggregates are concentrated in endosomes and lysosomes, and the amounts thereof gradually increase over time.

### <Example 4> Relationship between amyloid beta aggregation and PDE

From the result of Example 1, PDE3, which is specifically highly expressed in pericytes, is selected as a target material. It was observed how the treatment of cilostazol known as a specific inhibitor of PDE3 affects amyloid beta accumulation.

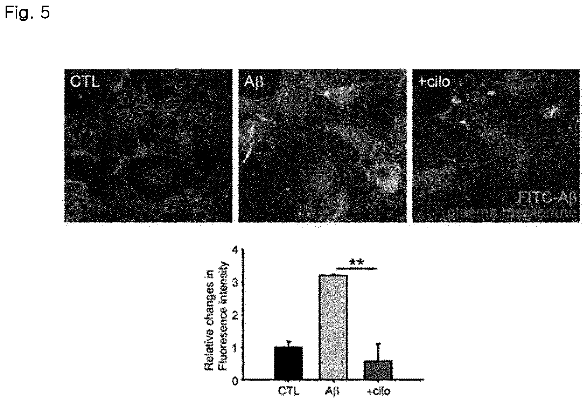

FIG. 5 is a set of confocal microscope images of mouse pericytes which are cultured and then treated with CTL (control), amyloid beta (Aβ) and cilostazol (amyloid beta+cilostazol; +cilo) for 1 hour, respectively. The graph on the right side of FIG. 5 is obtained by observing pericytes treated with CTL (control), amyloid beta (Aβ) and cilostazol (amyloid beta+cilostazol; +cilo) for 1 hour, and measuring the fluorescence intensities thereof.

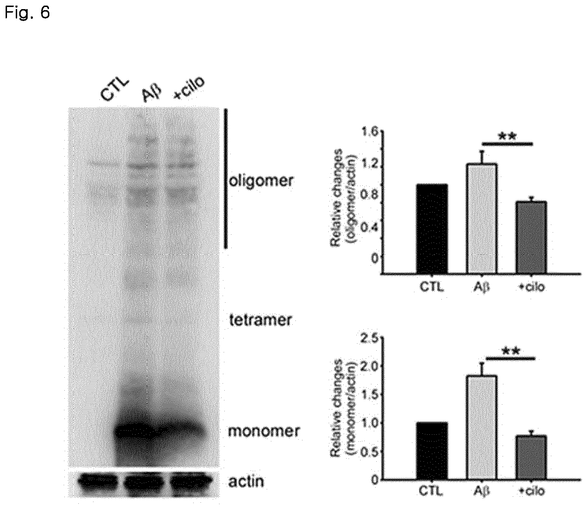

FIG. 6 shows a western blotting result for mouse pericytes which are cultured and then treated with CTL (control) or amyloid beta (Aβ) and cilostazol (amyloid beta+cilostazol; +cilo) for 24 hours, respectively. The graph on the right side of FIG. 6 is obtained by measuring an oligomer/actin expression level and a monomer/actin expression level in each group.

As shown in FIGS. 5 and 6, it was confirmed that citostazol significantly reduced the amount of aggregated amyloid beta. These results may prove the fact that PDE affects amyloid beta aggregation.

### <Example 5> Change in zinc concentration by PDE inhibitor

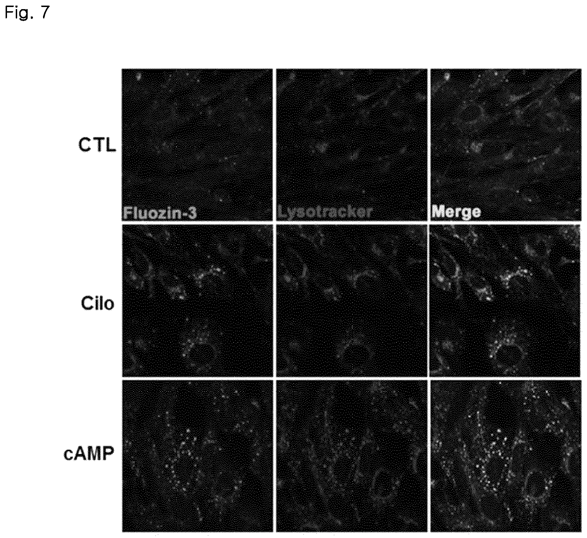

FIG. 7 is a set of images of mouse pericytes, which are cultured, treated with CTL (control), cilostazol (amyloid beta+cilostazol; +cilo) and cAMP (amyloid beta+cAMP) for 1 hour, and then stained with Fluozin-3 (staining zinc in the cytosol) and LysoTracker (staining zinc in acidic cell organelles), respectively.

As shown in FIG. 7, it can be confirmed that, when a PDE3 inhibitor is administered, or the concentration of cAMP is increased, the concentration of zinc in cells changes. The increase in intracellular zinc concentration in the cells treated with the PDE inhibitor or direct cAMP was able to be observed using a zinc dye, and it was found that the zinc concentration increases in lysosomes. The brain contains a large amount of zinc including synapse zinc, and when amyloid accumulation is reduced, the pH of lysosomes is changed by drugs that move extracellular zinc into cells, particularly, lysosomes, considering that zinc may play a pivotal role in lysosomal function.

### <Example 6> Change in pH of lysosomes

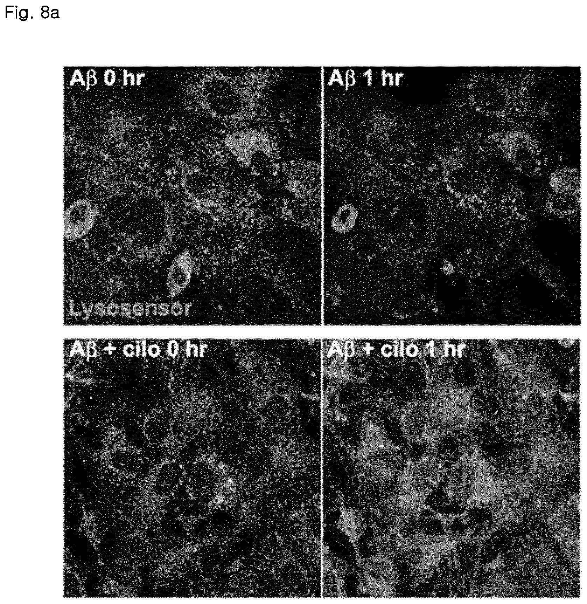

FIG. 8A is a set of images that visualize pH changes for 0 to 1 hour in respective groups of mouse astrocytes, which are stained with LysoSensor Green DND-189, and then treated with either amyloid beta (Aβ) or amyloid beta and cilostazol (Aβ+cilo).

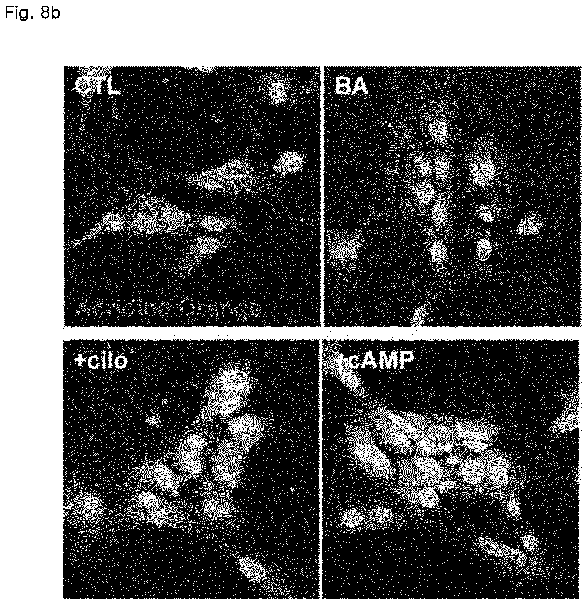

FIG. 8B is a set of images that visualize pH changes in respective groups of mouse astrocytes, which are stained with an acridine orange dye, and then treated with nothing (CTL; control), bafilomycin A1 (BA), bafilomycin+ cilostazol (+cilo) or bafilomycin + cAMP (+cAMP).

FIG. 8 shows the change in pH of lysosomes in PDE inhibitor-treated cells, obtained by an experiment for examining whether the effect of a PDE inhibitor is actually associated with the change in pH of lysosomes. It was confirmed that, in the amyloid beta-accumulated group, the pH of lysosomes is alkalized. On the other hand, in the cells to which cilostazol, which is a PDE3 inhibitor, or cAMP is directly administered, it was confirmed that pH is acidified, and the lysosomal function is also normalized.

In addition, like bafilomycin A, when a compound completely inhibiting vATPase is administered, it was confirmed that the same result was shown even under the condition of changing a lysosome pH in cells. That is, like modified presenilin 1, it is considered that the cAMP effect is likely to be exhibited even when the vATPase function is decreased.

### <Example 7> Construction of screening system and primary selection

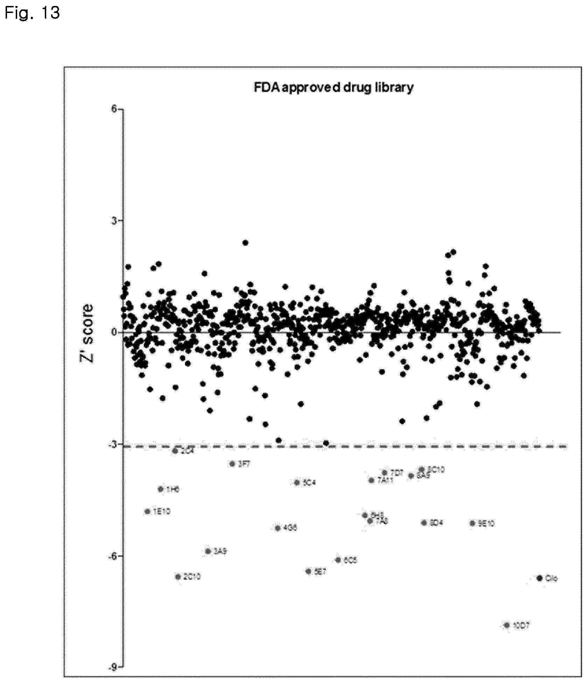

Libraries relevant to FDA-approved drugs that are currently used clinically and Selleckchem were obtained, and used in screening (FIG. 13). An experiment was performed by selecting only compounds which can be brought into Korea from the libraries.

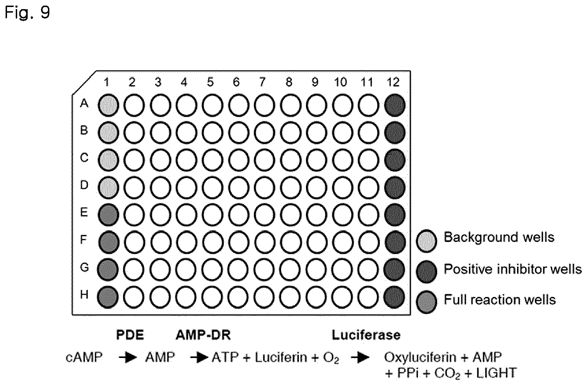

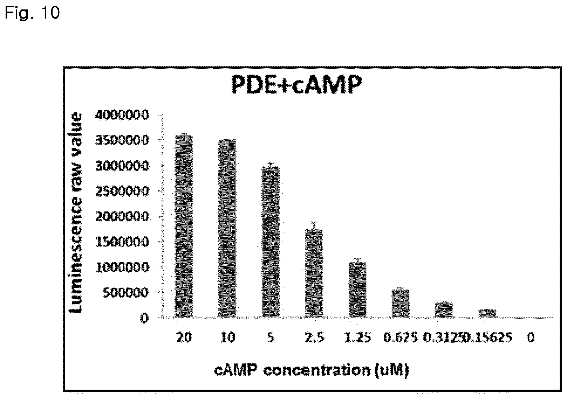

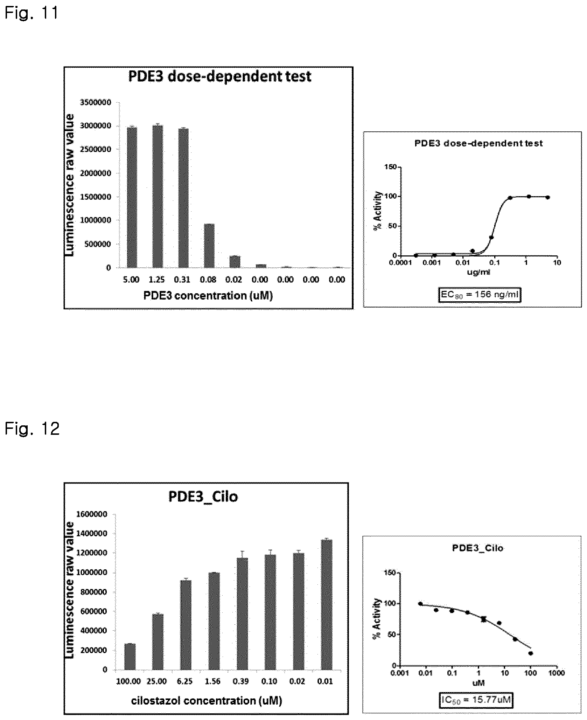

FIG. 9 shows the experimental method and device for HTS, FIG. 10 is a graph representing luminescence raw values according to cAMP concentrations in the presence of PDE and cAMP, and FIG. 11 shows the result of a PDE3 concentration-dependent test, represented by a set of graphs of luminescence raw values according to PDE3 concentrations and percent activity (% activity) according to PDE3 concentrations.

FIG. 12 is a set of graphs representing luminescence raw values for PDE3 according to cilostazol (positive control) concentrations and percent activity (% activity) according to the concentration of cilostazol, which is a PDE3 inhibitor.

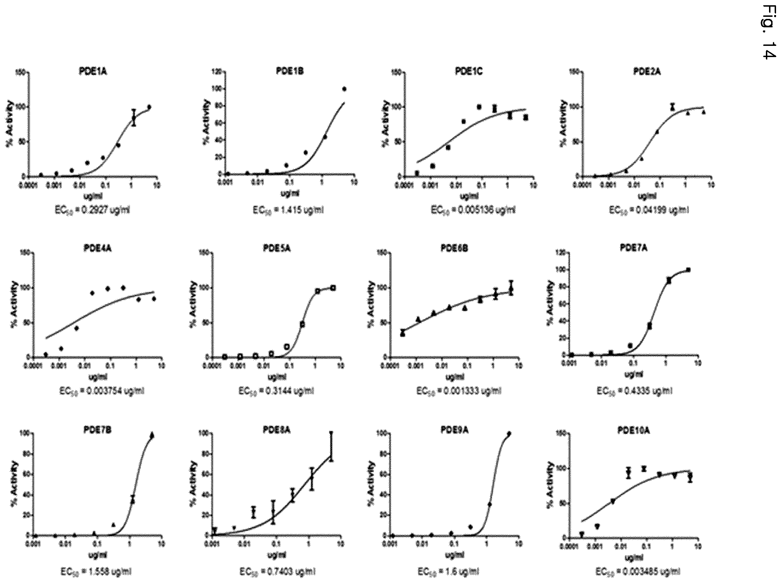

FIG. 13 is a graph representing Z' scores for the library of 800 FDA-approved drugs for PDE3, and FIG. 14 is a set of graphs for analyzing percent activity (% activity) according to concentrations of PDE isoforms.

Referring to FIGS. 9 to 14, before screening, the EC₅₀ for each PDE isoform was confirmed (FIG. 14), and an inhibitory concentration of a drug known as an inhibitor specific for the corresponding PDE isoform was ensured and used as a reference drug for comparison in screening.

In addition, as described above, in the present invention, to screen materials for treating neurodegenerative diseases such as Alzheimer's disease and Lou Gehrig's disease, among PDE isoforms, PDE3 was selected as a target, and an optimized concentration to be used in the experiment was selected to construct a drug screening system as shown in FIG. 9.

### <Example 8> Secondary selection

### Screening of cAMP activating material using HTS screening method

Candidate materials that increase cAMP activity were selected from the library of 800 FDA-approved drugs using a high throughput screening (HTS) method. Compounds included in the library of 800 FDA-approved drugs were diluted 1/4 from a concentration of 100 µM and dispensed into each well in 96-well plates, and mixed with a PDE reactant to allow a PDE reaction with the drug for 1 hour at a constant temperature. After a stop solution was mixed with an AMP detection agent, the mixture was dispensed at 20 µl per well to allow a reaction for 10 minutes, followed by immediately measuring luminescence (conducted as recommended by the PDELight HTS cAMP Phosphodiesterase Kit).

As a result of screening 800 drugs for PDE3 using the drug screening system constructed in Example 7, primarily, 21 compounds were selected. As a result of confirming IC₅₀, which is an inhibitory concentration of the 21 drugs, 11 compounds were secondarily selected.

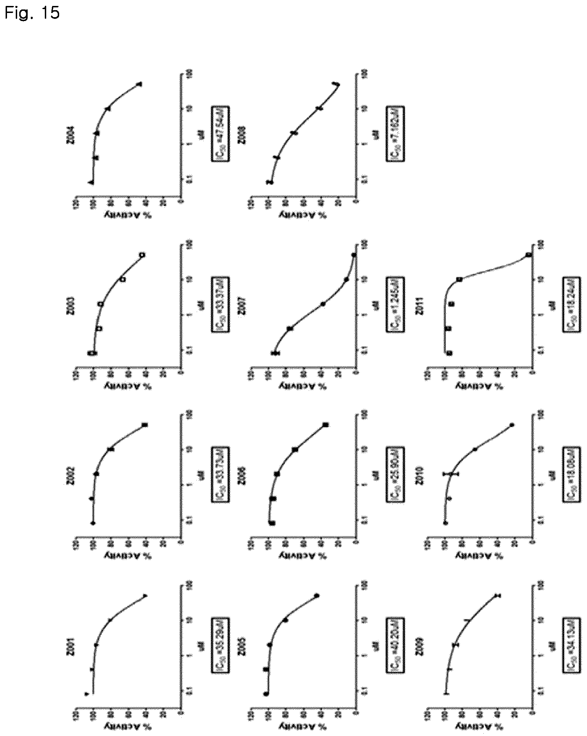

FIG. 15 is a set of graphs for analyzing percent activity (% activity) according to concentrations of primarily-selected compounds (Z001 to Z011) for PDE3. Referring to FIG. 15, it can be seen that Z007 (AMX) of the present invention exhibits the same or superior effect at a significantly lower concentration, which is at least 7 to 40-fold lower than other drugs, and has a significant inhibitory effect specific for PDE3, compared with conventional drugs.

### <Example 9> Analysis of amlexanox (AMX) effect on accumulation of various toxic proteins in vitro

For the 11 compounds selected according to Example 8, the HTS described in Example 7 was performed once again using all PDE isoforms as targets, and amlexanox (AMX; Z007) exhibiting excellent reducing efficacy for all PDE isoforms was finally selected.

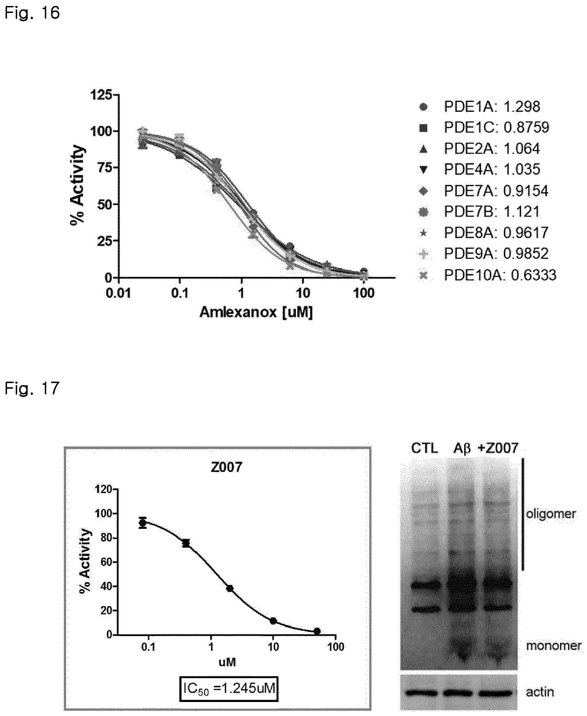

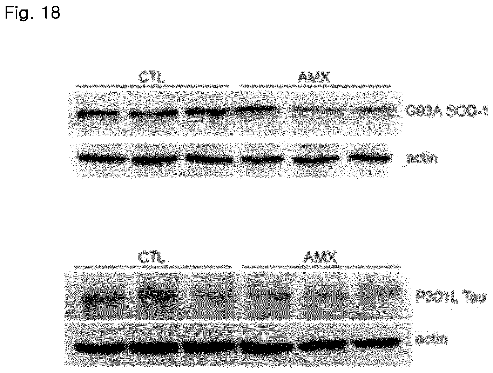

FIG. 16 is a graph representing the percent activity (% activity) according to the concentrations of finally selected amlexanox (AMX) for various PDE isoforms, and FIGS. 17 and 18 show western blotting images representing the amyloid beta expression pattern and G93A SOD-1 and P301L tau expression when amyloid beta-accumulated pericytes and astrocytes are treated with the finally selected amlexanox (AMX).

Referring to FIGS. 16 to 18, when amyloid beta was injected and accumulated in pericytes and astrocytes, and then the cells were treated with AMX, it was confirmed that the aggregation of an amyloid beta protein was inhibited. In addition, other than amyloid beta accumulation, it was confirmed that the accumulation of toxic proteins, such as Tau and G93A SOD-1, was also reduced by AMX.

### <Example 10> Analysis of amlexanox (AMX) effect on zinc concentration and lysosomal function in cells in vitro

A system that can visualize changes in intracellular lysosome pH with LysoSensor, which is a pH-sensitive lysosomal dye, in mouse pericytes using a confocal microscope was established (refer to Example 6) to observe whether amlexanox (AMX) is involved in the pH change of lysosomes in cells.

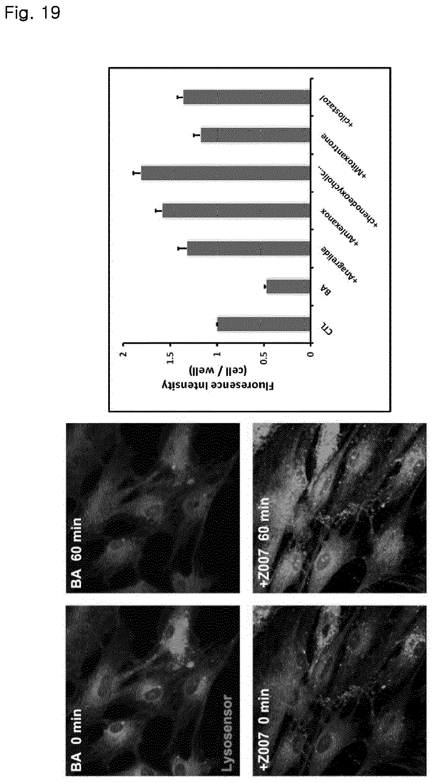

FIG. 19 is a set of time-dependent confocal microscope images (left) of mouse pericytes when being treated with Bafilomycin A1, which is a vATPase inhibitor, and then stained with LysoSensor, and quantified data (right) thereof.

Here, the images on the left side of FIG. 19 are obtained by treating mouse pericytes with bafilomycin A1, which is a vATPase inhibitor, and analyzing the cells using LysoSensor over time by a confocal microscope (top), and by treating the bafilomycin A1-treated mouse pericytes with the finally selected amlexanox (AMX) and analyzing the cells using LysoSensor over time by a confocal microscope (bottom).

As shown in FIG. 19, it was confirmed that lysosomal pH is increased by the treatment of the mouse pericytes with bafilomycin A1, and changed to alkaline. That is, it was able to confirmed that the bafilomycin A1 worsens (alkalizes) the pH environment of lysosomes in cells, but by the HTS system of the present invention, the finally selected amlexanox restores (acidifies) the lysosome pH environment of pericytes, which has changed to alkaline due to bafilomycin A1, to an original state within 60 minutes.

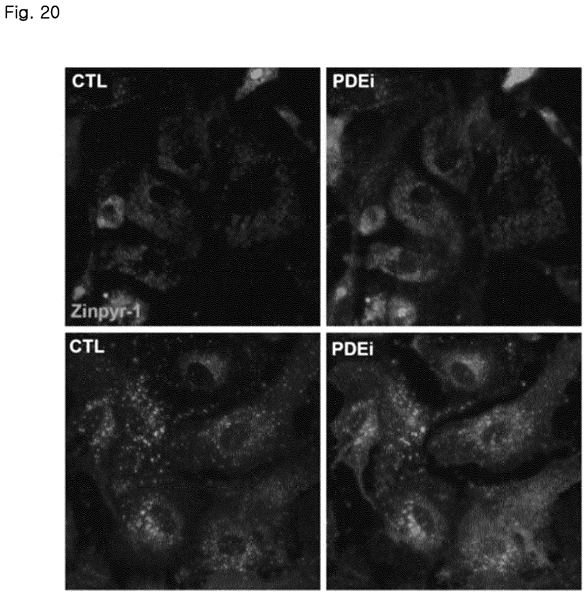

FIG. 20 is a set of confocal microscope images of groups of mouse pericytes, which are not treated (CTL) or treated with finally-selected amlexanox (AMX) (PDEi), in which, to measure a zinc concentration, fluorescence intensity was detected by treating the cells with a fluorescent dye (Zinpyr-1) for detecting zinc ions.

As shown in FIG. 20, when amlexanox (AMX) is administered into the pericytes, it was confirmed that the concentration of zinc in the cell is restored and increased to a normal level.

### <Example 11> Study of verifying lysosomal function activity

Astrocytes were stained with a fluorescence-tagged lysosomal protein, and the degrees of hydrolysis of cathepsin B and cathepsin L, which are lysosomal proteins, were analyzed.

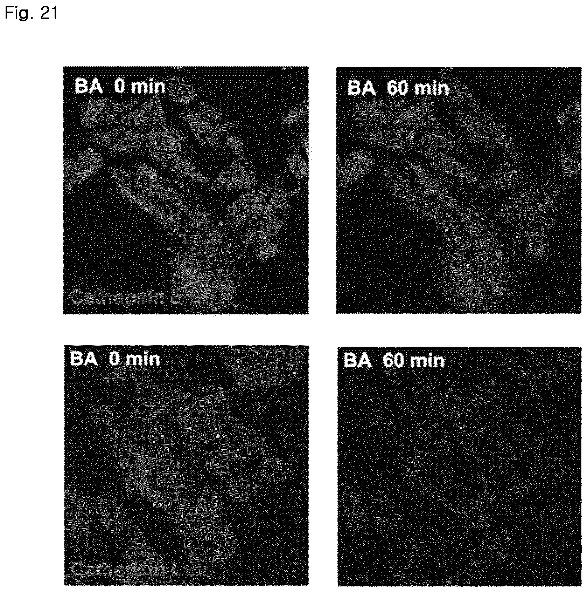

FIG. 21 is a set of time-dependent confocal microscope images of mouse astrocytes, which are treated with bafilomycin A1, which is a vATPase inhibitor, to assess the degree of hydrolysis of cathepsin B (top) and cathepsin L (bottom).

As shown in FIG. 21, in mouse astrocytes, it was confirmed that, as bafilomycin A1 was treated, cathepsin L and cathepsin B, which are lysosomal proteins, decreased, indicating that bafilomycin A1 significantly degrades a lysosomal function in astrocytes. In other words, it was confirmed that BA reduces the activity of both of the lysosomal proteins.

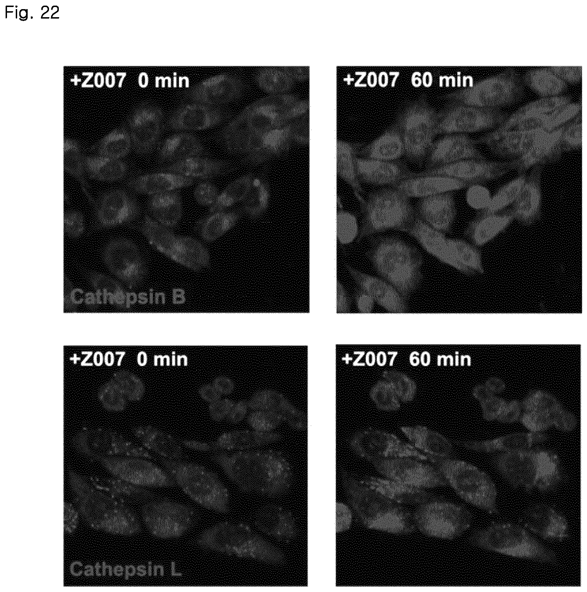

FIG. 22 is a set of time-dependent confocal microscope images of bafilomycin A1-treated mouse astrocytes, which are treated with finally selected amlexanox (AMX; Z007), to assess the degree of hydrolysis of cathepsin B (top) and cathepsin L (bottom), confirming that the lysosomal protein activity and function in the astrocytes were degraded by bafilomycin A1, and the amlexanox (AMX) treatment suppressed the effect of bafilomycin A1, thereby restoring the activity and function of the lysosomal proteins in the astrocytes to a normal level.

That is, it was able to be confirmed that when the amlexanox is treated, the lysosomal activity of cathepsin B or cathepsin L in lysosomes of the astrocytes is restored to an original state within 60 minutes.

### <Example 12> Analysis of preventive or therapeutic effect of amlexanox in Alzheimer's disease animal models

To confirm whether amlexanox (AMX; Z007) is effective even for animal models, an experiment was conducted using 5X FAD mice, which is one of the Alzheimer-type dementia animal models. Here, the 5X FAD mice, which is one of the Alzheimer-type dementia animal models, were provided in Korea and used.

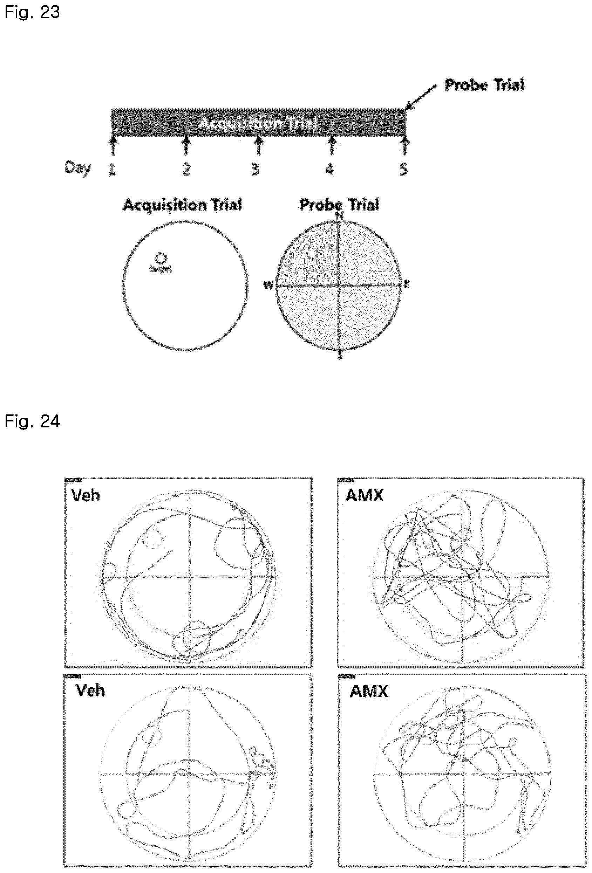

Drug administration and behavioral evaluation were conducted on the same schedule as in FIG. 23, and specifically, amlexanox (AMX; Z007) was dissolved in DMSO and intraperitoneally administered into 3-month-old 5X FAD female mice at a dose of 1 mg/kg once a day for 3 months. Immediately after the end of the administration, a Morris water maze test, which is a behavioral experiment that can evaluate memory and learning ability, was conducted.

The Morris water maze test was performed as described in Behav. Brain Res. (155, 185-196) (refer to Behav. Brain Res., 155, 185-196). A water tank used in the water maze test is a cylindrical tank with a diameter of 90 cm and a height of 60 cm, and filled with water in which milk powder is diluted at 23 ± 2 °C during a test period. In the tank, a transparent platform was installed 1 cm below the water surface, and four signs were placed outside the tank. The motion trajectory of a mouse was analyzed through a video tracking system (Smart3.0, Panlab, USA).

During the trial period, the mice were placed into the water tank at four randomly selected starting points toward the side where the platform was not shown. In each trial, an escape latency, which is the time to find the platform after the mouse is put into the water tank, was recorded, and when finding the platform, the mouse was allowed to stay on the platform for 10 seconds and then moved to a home cage. When the mouse did not find the platform within 60 seconds, the escape latency was recorded as 60 seconds. Each trial was performed four times a day for 5 days.

A probe test was conducted on the last 5 days of the test. After the platform was removed, the test was performed by allowing a mouse to swim in a water tank for 60 seconds and recording the time to first enter the location of the platform and how many times the mouse passed the location.

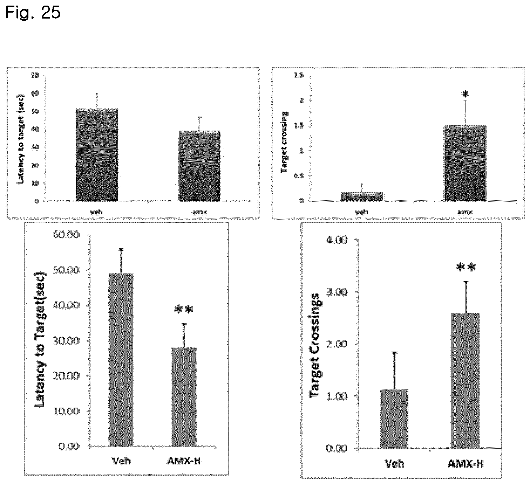

FIG. 23 is a diagram illustrating the test schedule for evaluating the pharmacological efficacy of amlexanox (AMX) in Alzheimer's disease animal models, 5X FAD ((Swedish mutation (KM670/671NL: an increase in Aβ production), Florida mutation (I716V: an increase in Aβ42 production), London mutation (V717I: an increase in Aβ42 production), human presenilin 1 (M146L and L286V: an increase in Aβ42 production) mice). FIGS. 24 and 25 are a set of graphs representing the pharmacological efficacy of amlexanox (AMX) on a decrease in cognitive memory by amyloid beta (Aβ1-42) and PS1 overexpression, analyzed by water maze evaluation consisting of a hidden platform test and a probe test. FIG. 24 (top: 1 mg/kg, bottom: 2.5 mg/kg) shows the movement path of an animal model searching for a target in each test group (Veh group (control) and AMX-administered group).

As shown in FIGS. 24 and 25 (top: 1 mg/kg, bottom: 2.5 mg/kg), it was able to confirm that, in animal models in the AMX-administered group (amx), compared with animal models in the Veh (vehicle) group (control), the time to find the target point does not only increase, but the number of passing the target point also increases. Therefore, it can be seen that the amlexanox (AMX)-administered Alzheimer's disease animal models are improved in memory and learning ability.

### <Example 13> Analysis of preventive or therapeutic effect of amlexanox in Lou Gehrig's diseases animal models

G93A SOD-1 mice in which a mutant protein of the ALS-related gene SOD-1 (the expression of an amino acid at codon 93, in which glycine is substituted with alanine) of a human were used as Lou Gehrig's disease animal models. The G93A SOD-1 mice were purchased by the Jackson Laboratory (USA).

In the animal models of Lou Gehrig's disease, spinal motor neuron death occurs, causing limb paralysis at one or both sides. The clinical scoring system was differentiated using the following neurological scoring system.
Normal without sign of motor disorder: 4 points
Hind paw trembling during tail suspension: 3 points
Abnormal gait: 2 points
Dragging at least one hindlimb: 1 point
Inability to correct itself within 30 seconds: 0 points

The mice were euthanized at 0 points (Weydt et al. 2003). In the Lou Gehrig's disease animal models, which is one of the neurodegenerative disease models, a vehicle or amlexanox (AMX) was intraperitoneally administered at 2.5 mg/kg once daily for 5 days, and then a survival rate was measured through a behavioral experiment.

The behavioral experiment was performed on each of the AMX-administered group and the control (Veh) once or twice a week before symptoms occur until death. A method of gradually increasing a speed from 4 rpm to 40 rpm and a method of exercising at a constant speed of 15 rpm for 10 minutes were selected. The time to fall off from a rod was recorded to analyze the difference between the AMX-administered group and the control group.

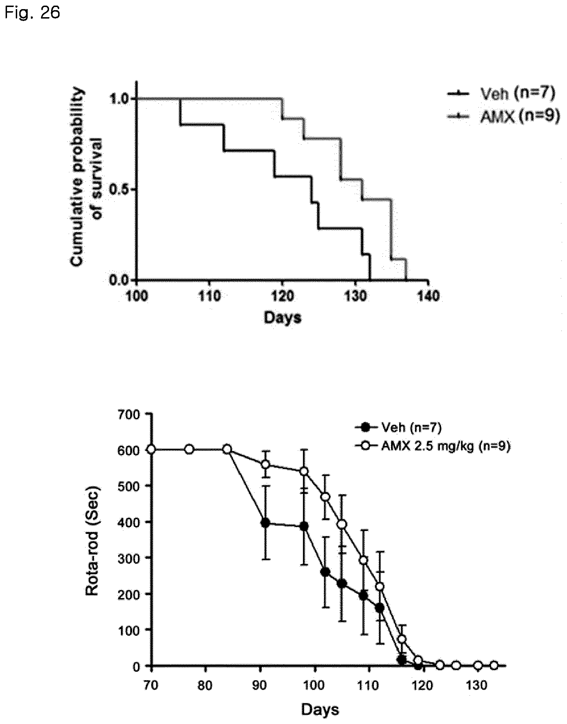

FIG. 26 shows the results of experiments performed by dividing Lou Gehrig's disease animal models (G93A SOD-1 mice) into two groups (AMX-treated group and control (Veh)), and each of the vehicle and amlexanox was intraperitoneally administered into G93A SOD-1 mice at 2.5 mg/kg once daily for 5 days a week from week 10 until the neurological score was 0, and then the survival rate was compared. It was confirmed that the survival rate of the AMX administered group (130.2±2.0 days) was significantly increased, compared with that of the control (veh) (121.3±3.6 days). That is, when amlexanox (AMX) was administered into the Lou Gehrig's disease animal model, it was confirmed that the effect of prolonging the time of onset of Lou Gehrig's disease and survival rate thereof may be obtained, and according to such results, it can be seen that amlexanox (AMX), which has been newly suggested by the inventors, is effective in consistent prevention of the aggravation of motor symptoms in the process of a neurodegenerative disease such as Lou Gehrig's disease or Alzheimer's disease.

### <Example 14> Analysis of cell protective effect of AMX on cell death

In terms of cell damage generated by accumulation of a toxic protein by amyloid beta, the cell protective effect of amlexanox (AMX) on astrocytes was confirmed. That is, since the accumulation of a toxic protein caused by amyloid beta causes oxidative damage to cells, leading to cell death, the pharmacological efficacy of AMX was confirmed.

Specifically, cell death caused by oxidative damage was induced by injecting H₂O₂ into astrocytes, a cell culture model similar to the animal model with a neurodegenerative disease such as Alzheimer's disease or Lou Gehrig's disease was manufactured. To confirm the cell protective effect of amlexanox (AMX) in the cell model manufactured as described above, an untreated H₂O₂ control and an experimental group produced by administering amlexanox (AMX) into the untreated H₂O₂ control were prepared, and after 24 hours, cell death was quantitatively assessed by a LDH method.

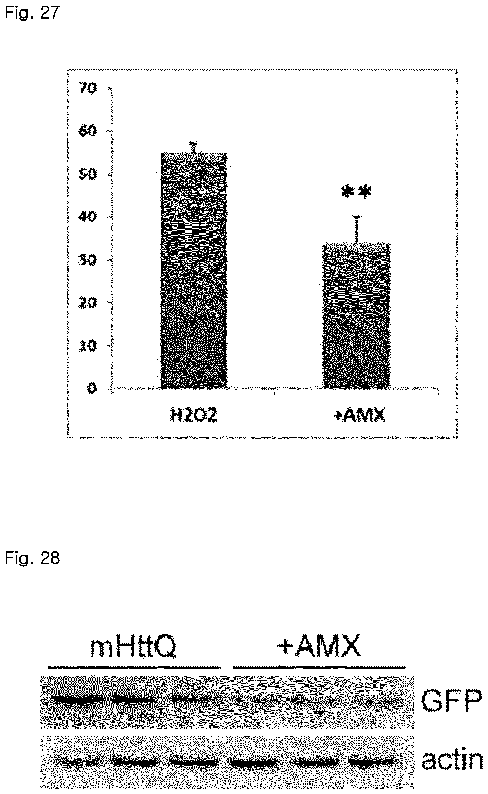

FIG. 27 is a graph obtained by inducing cell death by causing oxidative damage to astrocytes with H₂O₂, and treating the cells with amlexanox (AMX) to quantify the cell death using an LDH method. Accordingly, it was confirmed that, in the amlexanox (AMX)-treated experimental group, cell death was significantly reduced up to 40%, and in the control, the cell death was significantly increased up to 60%.

### <Example 15> GFP-mHttQ aggregation inhibitory effect of AMX

A GFP-tagged mHttQ protein was injected into cells, thereby confirming a pharmacological reaction mediated by AMX using a GFP antibody. The accumulation of mHttQ in cells through aggregation was confirmed by GFP expression.

As a result, as shown in FIG. 28, the aggregated protein-inhibitory effect of AMX was reconfirmed through the decrease in GFP expression.

### <Example 16> Improvement in survival rate of AMX, G93A-SOD mouse models

Changes in survival rate were confirmed by intraperitoneal injection into G93A-SOD mice daily at various AMX concentrations.

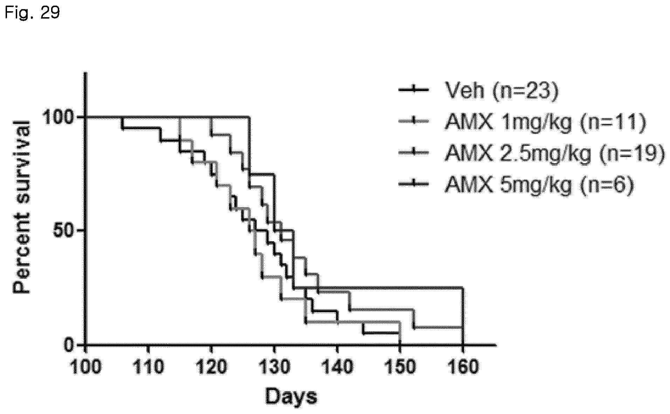

From the result shown in FIG. 29, which is the same as that of FIG. 26, it was confirmed that the untreated Veh group shows a survival rate of approximately 150 days, whereas in the AMX-treated group, the survival rate was concentration-dependently increased.

### <Example 17> Effect of delaying the progression of disease by AMX in G93A-SOD mouse model

As well as the survival rate improving effect described in Example 15, the concentration-dependent changes in ataxia caused by AMX were confirmed.

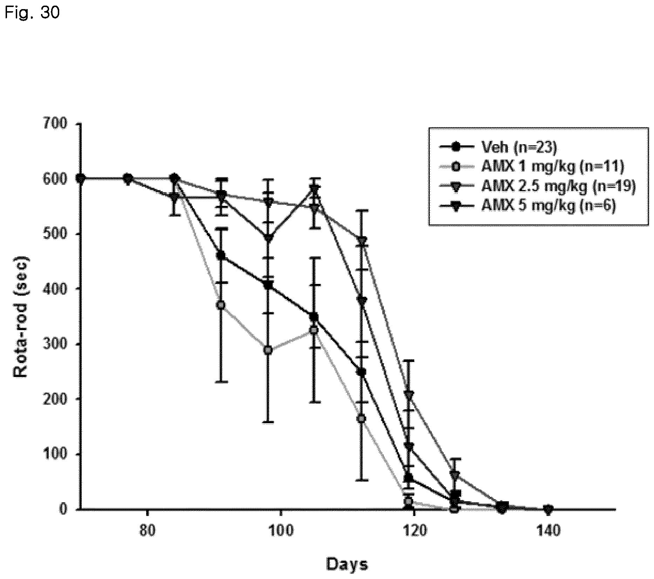

From the result shown in FIG. 30, which is similar to that of FIG. 26, it was confirmed that, in the untreated control, ataxia rapidly and aggressively develops, whereas in the groups in which AMX is injected at various concentrations, the onset of ataxia is slower, and its progression is also reduced.

### <Example 18> Screening of PDE inhibitor

For drug efficacy screening for PDE inhibition, HTS was used. cAMP-based screening was conducted with an FDA-approved drug library and a Selleckchem library based on the conventional PDE inhibitor. The experiment was carried out in the same manner as used in screening of the FDA-approved drug library. Compared with cilostazol, which is known as a PDE3 inhibitor, drugs with high inhibitory activity were selected. This is to select drugs increasing cAMP activity from among the drugs to be screened. Each of the screening library drugs were diluted 1/4 from 100 µM and dispensed into each well in 96-well plates, and mixed with a PDE reactant, followed by allowing a reaction between the drug and PDE for 1 hour for storage. After a stop solution was mixed with a AMP detection agent, the mixture was dispensed at 20 µl per well to allow a reaction for 10 minutes, followed by immediately measuring luminescence, and the experiment was performed according to a protocol provided by the kit used herein.

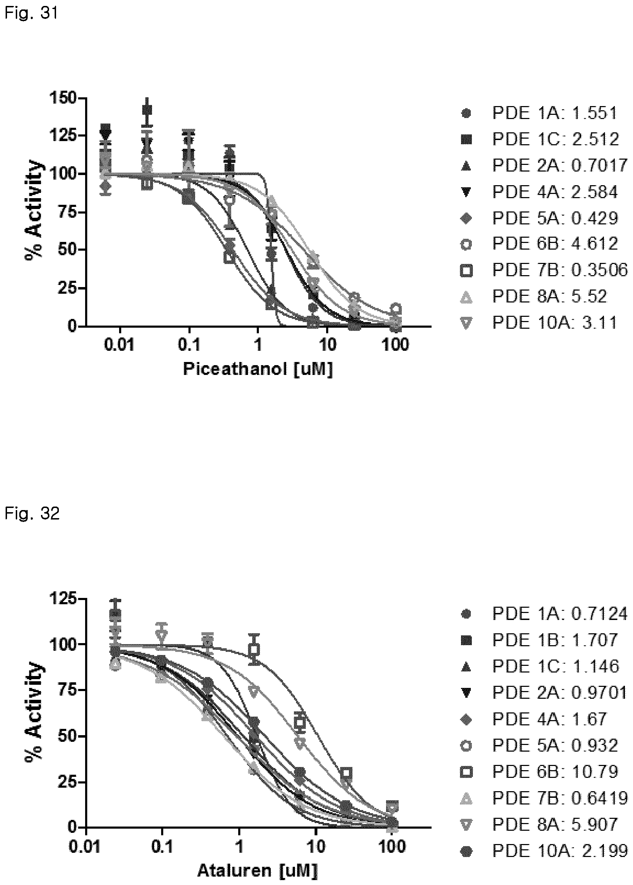

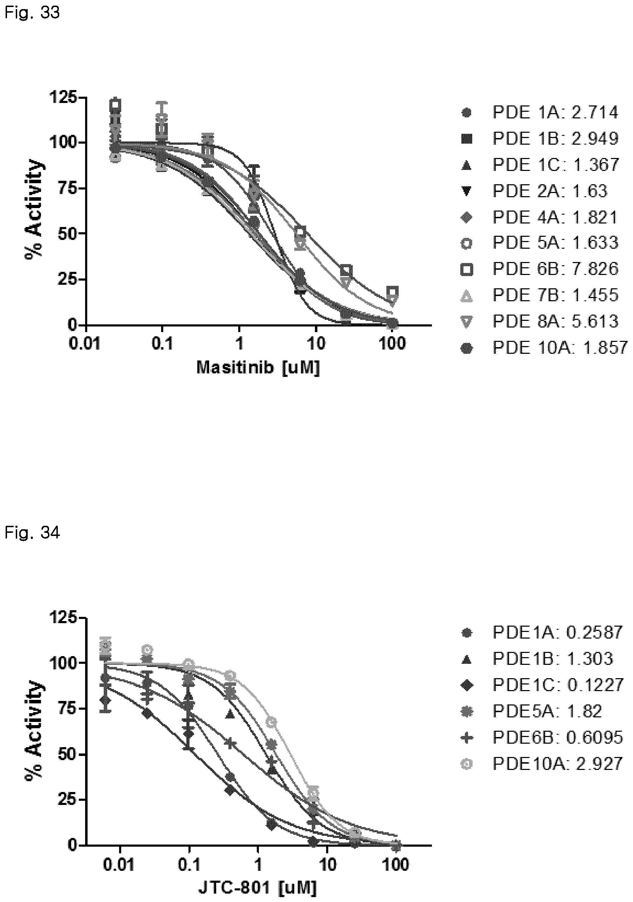

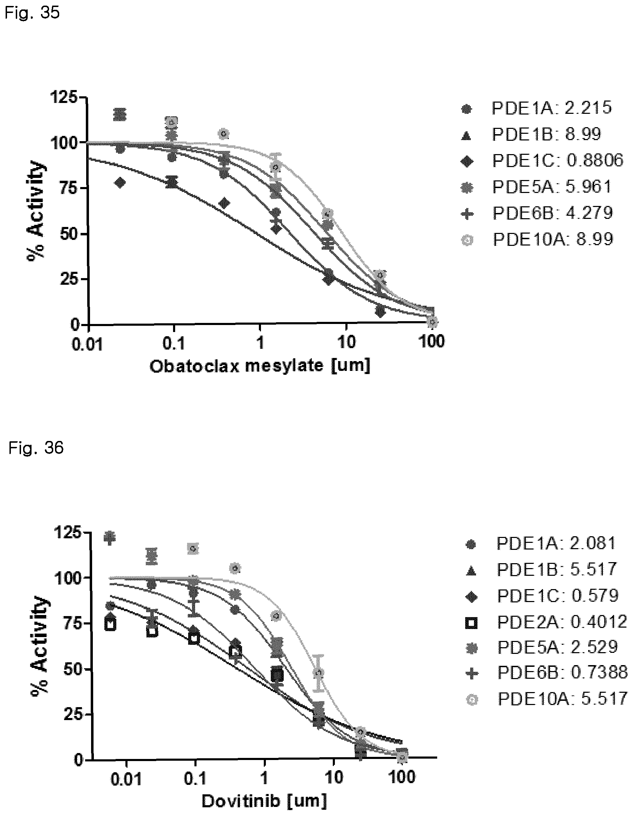

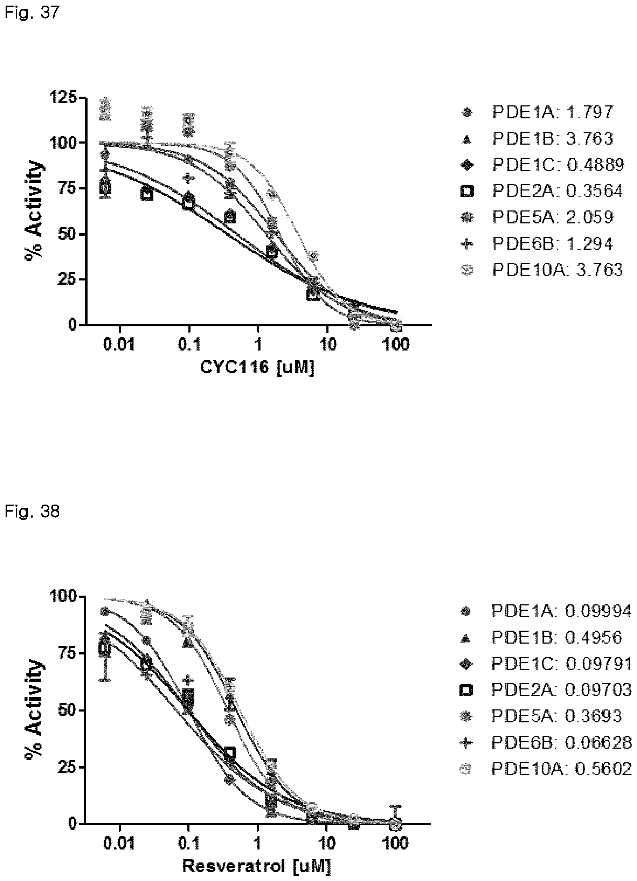

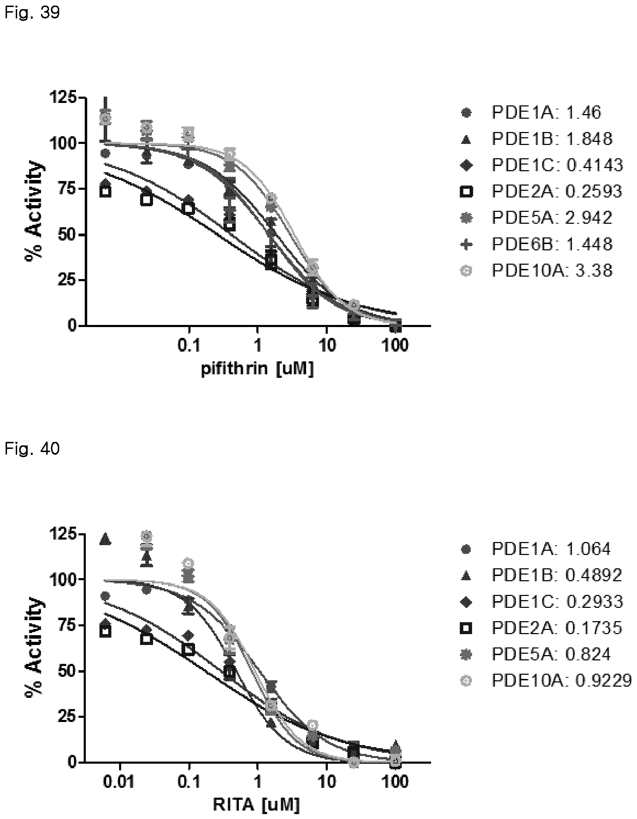

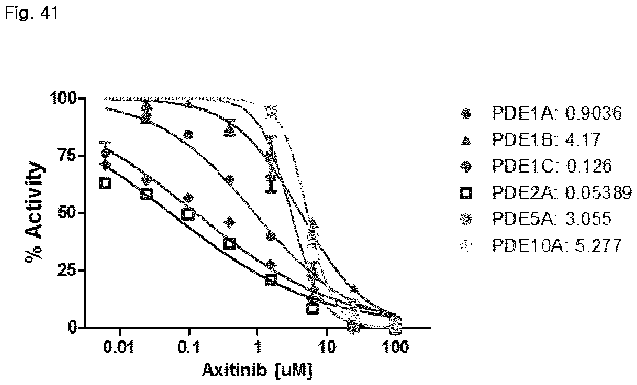

The first selection started with PDE3 as a target, and after candidate drugs were selected, all PDE isoforms were tested. 11 drugs showing high inhibitory efficacy in all PDE isoforms were selected, and illustrated in FIGS. 31 to 41 (11 drugs: piceathanol, ataluren, masitinib, JTC-801, obatoclax mesylate, dovitinib, CYC116, resveratrol, pifithrin), 5,5'-(2,5-furandiyl)bis-2-thiophenemethanol (RITA), axitinib, imatinib mesylate, zafirlukast, hexachloropene, febuxostat and silver sulfadiazine, listed in Table 1 in this specification).

As shown in FIG. 31, piceatanol is a drug having the best PDE inhibitory value, and as shown in the graph, the inhibition value of each isoform is significantly lowered to at least 0.3 nM and a maximum of 5.5 µM. It was confirmed that the inhibition values of the all PDE isoforms of the other drugs were also excellent.

That is, it can be clearly confirmed that the PDE inhibitor, particularly, amlexanox (AMX) plays a very important role in cell death in cells (astrocytes, neurons and pericytes) with oxidative damage caused by the accumulation of amyloid beta due to a neurodegenerative disease such as Alzheimer's disease or Lou Gehrig's disease, and has a pharmacological effect of restoring or preventing cell damage.

As above, as specific parts of the specification have been described in detail, although it is clear to those skilled in the art that this specific technique is merely a preferred embodiment, the scope of the specification is not limited thereto. Thus, the substantial scope of the specification will be defined by the accompanying claims and their equivalents.

### [Industrial Applicability]

The composition of the present invention can be effectively used in safe and effective prevention or treatment of an amyloid beta-caused neurodegenerative disease and cell damage without concerns about side effects, and thus has industrial applicability.

## Claims

1. A pharmaceutical composition for preventing or treating a neurodegenerative disease, comprising:
a phosphodiesterase (PDE) inhibitor or a pharmacologically acceptable salt thereof as an active ingredient.

2. A pharmaceutical composition for preventing or treating a neurodegenerative disease, comprising:
amlexanox (AMX) or a pharmacologically acceptable salt thereof as an active ingredient.

3. A pharmaceutical composition for preventing or treating a neurodegenerative disease, comprising:
one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient.

4. The pharmaceutical composition of claim 1, wherein the phosphodiesterase (PDE) is one or more selected from the group consisting of PDE1A, PDE1B, PDE1C, PDE2A, PDE3A, PDE3B, PDE4A, PDE4B, PDE5A, PDE6A, PDE6B, PDE6C, PDE6D, PDE7A, PDE7B, PDE8A, PDE8B, PDE9A, PDE10A and PDE11A.

5. The pharmaceutical composition of claim 2, wherein the amlexanox or a pharmacologically acceptable salt thereof prevents or reduces the aggregation of toxic proteins caused by amyloid beta accumulation.

6. The pharmaceutical composition of claim 2, wherein the amlexanox or a pharmacologically acceptable salt thereof prevents or reduces cell death caused by oxidative stress.

7. The pharmaceutical composition of claim 2, wherein the pharmacologically acceptable salt is an acid-addition salt formed by an organic acid selected from oxalic acid, maleic acid, fumaric acid, malic acid, tartaric acid, citric acid and benzoic acid, or an inorganic acid selected from hydrochloric acid, sulfuric acid, phosphoric acid and hydrobromic acid.

8. The pharmaceutical composition of any one of claims 1 to 3, wherein the neurodegenerative disease is selected from the group consisting of Alzheimer's disease (AD), Parkinson's disease (PD), Huntington's disease, Lou Gehrig's disease (ALS), posttraumatic stress disorder (trauma), multiple sclerosis (MS), cerebral ischemic disease and amyotrophic lateral sclerosis.

9. The pharmaceutical composition of claim 8, wherein the neurodegenerative disease is Alzheimer's disease.

10. A food composition for preventing or improving a neurodegenerative disease, comprising:
a phosphodiesterase (PDE) inhibitor or a sitologically acceptable salt thereof as an active ingredient.

11. A food composition for preventing or improving a neurodegenerative disease, comprising:
amlexanox or a sitologically acceptable salt thereof as an active ingredient.

12. A food composition for preventing or improving a neurodegenerative disease, comprising:
one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a sitologically acceptable salt thereof as an active ingredient.

13. A pharmaceutical composition for preventing or treating cell damage, comprising:
amlexanox or a pharmacologically acceptable salt thereof as an active ingredient.

14. The pharmaceutical composition of claim 13, wherein the cell damage is an increase in lysosome pH by amyloid beta, and a decrease in activity of a lysosomal protein or zinc concentration in cells.

15. The pharmaceutical composition of claim 13, wherein the cells are any one or more selected from the group consisting of pericytes, neurons and astrocytes.

16. A food composition for preventing or improving cell damage, comprising:
amlexanox or a sitologically acceptable salt thereof as an active ingredient.

17. A use of a phosphodiesterase (PDE) inhibitor or a pharmacologically acceptable salt thereof as an active ingredient to prevent or treat a neurodegenerative disease.

18. A use of amlexanox or a pharmacologically acceptable salt thereof as an active ingredient to prevent or treat a neurodegenerative disease.

19. A use of one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof as an active ingredient to prevent or treat a neurodegenerative disease.

20. A method of preventing or treating a neurodegenerative disease by administering a phosphodiesterase (PDE) inhibitor or a pharmacologically acceptable salt thereof into an individual.

21. A method of preventing or treating a neurodegenerative disease by administering amlexanox or a pharmacologically acceptable salt thereof into an individual.

22. A method of preventing or treating a neurodegenerative disease by administering one or more compounds selected from the group consisting of piceathanol, ataluren, masitinib, JTC-801, obatoclax mesylate, dovitinib, CYC116, resveratrol, pifithrin, 5,5'-(2,5-furandiyl)bis-2-thiophenemethanol (RITA), axitinib, imatinib mesylate, zafirlukast, hexachloropene, febuxostat, and silver sulfadiazine, or a pharmacologically acceptable salt thereof into an individual.


<110>    THE ASAN FOUNDATION
         University of Ulsan Foundation For Industry Cooperation

<120>    A Composition for Prevention or Treatment of Neurodegenerative
         disease

<130>    MPCT19-078

<150>    KR 10-2018-0071564
<151>    2018-06-21

<150>    KR 10-2019-0074410
<151>    2019-06-21

<160>    66

<170>    KoPatentIn 3.0

<210>    1
<211>    25
<212>    DNA
<213>    Artificial Sequence

<220>
<223>    PDE1A-F


<400>    1
gaagcagctg gagaaaggtg atgtc                                              25


<210>    2
<211>    25
<212>    DNA
<213>    Artificial Sequence

<220>
<223>    PDE1A-R


<400>    2
cctttccaca aaaatcccag cctgg                                              25


<210>    3
<211>    25
<212>    DNA
<213>    Artificial Sequence

<220>
<223>    PDE1B-F


<400>    3
gcgctacatg gtgaagcagt tggag                                              25


<210>    4
<211>    22
<212>    DNA
<213>    Artificial Sequence

<220>

<223>    PDE1B-R

<400>    4
ccacgaagat cccagcctgc ac                                                22

<210>    5
<211>    24
<212>    DNA
<213>    Artificial Sequence

<220>
<223>    PDE1C-F

<400>    5
cggctgccat ccatgactat gaac                                              24

<210>    6
<211>    24
<212>    DNA
<213>    Artificial Sequence

<220>
<223>    PDE1C-R

<400>    6
gttcgaaact ccctccagtc atcc                                              24

<210>    7
<211>    23
<212>    DNA
<213>    Artificial Sequence

<220>
<223>    PDE2A-F

<400>    7
ggtcataccc ctgctggaca agg                                               23

<210>    8
<211>    25
<212>    DNA
<213>    Artificial Sequence

<220>
<223>    PDE2A-R

<400>    8
cagttgcagg atctttcggt catgg                                             25

<210>    9
<211>    25
<212>    DNA
<213>    Artificial Sequence

<220>
<223>    PDE3a F

<400>    9
actttcatgc tttggagatc ggata                                          25

<210>    10
<211>    22
<212>    DNA
<213>    Artificial Sequence

<220>
<223>    PDE3a R

<400>    10
aggaatcggc tgtgttgtga ga                                             22

<210>    11
<211>    24
<212>    DNA
<213>    Artificial Sequence

<220>
<223>    PDE3B-F

<400>    11
ctctgggact gggacttgaa gcag                                           24

<210>    12
<211>    26
<212>    DNA
<213>    Artificial Sequence

<220>
<223>    PDE3B-R

<400>    12
ctttggccta caggaacctg agaaag                                         26

<210>    13
<211>    22
<212>    DNA
<213>    Artificial Sequence

<220>
<223>    PDE4A-F

<400>    13
gcttggaggc agaaaatggg cc                                             22

<210>    14
<211>    20

<212>    DNA
<213>    Artificial Sequence

<220>
<223>    PDE4A-R


<400>    14
cggagactgg ccagcacctg                                                    20


<210>    15
<211>    25
<212>    DNA
<213>    Artificial Sequence

<220>
<223>    PDE4B-F


<400>    15
gtggaaaatg gcccttctcc aggtc                                              25


<210>    16
<211>    27
<212>    DNA
<213>    Artificial Sequence

<220>
<223>    PDE4B-R


<400>    16
ggcaaaagga gtgacaatca ggtcatc                                            27


<210>    17
<211>    26
<212>    DNA
<213>    Artificial Sequence

<220>
<223>    PDE5A-F


<400>    17
cgcctctttg atgttgctga aggttc                                             26


<210>    18
<211>    26
<212>    DNA
<213>    Artificial Sequence

<220>
<223>    PDE5A-R


<400>    18
cctgatttct tgttgatggc ctgagc                                             26

<210>    19
<211>    24
<212>    DNA
<213>    Artificial Sequence

<220>
<223>    PDE6A-F


<400>    19
gatgtgtggc cagttctgat gggc                                              24


<210>    20
<211>    25
<212>    DNA
<213>    Artificial Sequence

<220>
<223>    PDE6A-R


<400>    20
catccagagg ctctttctgg aattc                                             25


<210>    21
<211>    25
<212>    DNA
<213>    Artificial Sequence

<220>
<223>    PDE6B-F


<400>    21
gataaacgtg caggatgtgg cagag                                             25


<210>    22
<211>    25
<212>    DNA
<213>    Artificial Sequence

<220>
<223>    PDE6B-R


<400>    22
cacagttgtg gaggtagctt aggtg                                             25


<210>    23
<211>    25
<212>    DNA
<213>    Artificial Sequence

<220>
<223>    PDE6C-F


<400>    23
ccggagaagc cagatcctta tgtgg                                             25

<210>    24
<211>    24
<212>    DNA
<213>    Artificial Sequence

<220>
<223>    PDE6C-R


<400>    24
cctgccatct ggagtctttg gtcc                                              24


<210>    25
<211>    26
<212>    DNA
<213>    Artificial Sequence

<220>
<223>    PDE6D-F


<400>    25
gatgaacctt cgggatgccg aaacag                                            26


<210>    26
<211>    26
<212>    DNA
<213>    Artificial Sequence

<220>
<223>    PDE6D-R


<400>    26
gccaggtgtt tgtggagtta gggatc                                            26


<210>    27
<211>    22
<212>    DNA
<213>    Artificial Sequence

<220>
<223>    PDE7A-F


<400>    27
cagctctcgc agagacgtgg ag                                                22


<210>    28
<211>    26
<212>    DNA
<213>    Artificial Sequence

<220>
<223>    PDE7A-R

<400>    28
ccagcataca cttggcttgt ccattg                                            26


<210>    29
<211>    27
<212>    DNA
<213>    Artificial Sequence

<220>
<223>    PDE7B-F


<400>    29
gttgtgtcac ctcttcaact cccatgg                                           27


<210>    30
<211>    23
<212>    DNA
<213>    Artificial Sequence

<220>
<223>    PDE7B-R


<400>    30
gcatgatgtc cagaggtgtg agg                                               23


<210>    31
<211>    24
<212>    DNA
<213>    Artificial Sequence

<220>
<223>    PDE8A-F


<400>    31
gtcaccaagg aggtgcagac tgtc                                              24


<210>    32
<211>    26
<212>    DNA
<213>    Artificial Sequence

<220>
<223>    PDE8A-R


<400>    32
gagttacaag ccctgagttt cagctg                                            26


<210>    33
<211>    27
<212>    DNA
<213>    Artificial Sequence

<220>
<223>    PDE8B-F

<400>    33
cagtgtttac agcgttagac cactgtc                                          27


<210>    34
<211>    27
<212>    DNA
<213>    Artificial Sequence

<220>
<223>    PDE8B-R


<400>    34
cgaatgaggc tctgtctgag agttgtc                                          27


<210>    35
<211>    22
<212>    DNA
<213>    Artificial Sequence

<220>
<223>    PDE9A-F


<400>    35
gacacctcga cgtgatgtcc cc                                               22


<210>    36
<211>    25
<212>    DNA
<213>    Artificial Sequence

<220>
<223>    PDE9A-R


<400>    36
catctgtgtc acacagaagc agtgc                                            25


<210>    37
<211>    26
<212>    DNA
<213>    Artificial Sequence

<220>
<223>    PDE10A-F


<400>    37
gggattgctg gtcaagtggc aagaac                                           26


<210>    38
<211>    27
<212>    DNA
<213>    Artificial Sequence

<220>
<223>    PDE10A-R


<400>    38
gcttctccat ggtaaccctg tagatgc                                          27


<210>    39
<211>    22
<212>    DNA
<213>    Artificial Sequence

<220>
<223>    PDE11A-F


<400>    39
gctggaggtg gtcaatgacc tc                                               22


<210>    40
<211>    23
<212>    DNA
<213>    Artificial Sequence

<220>
<223>    PDE11A-R


<400>    40
ctgtcgaagc aaccagctca gcg                                              23


<210>    41
<211>    26
<212>    DNA
<213>    Artificial Sequence

<220>
<223>    GAPDH-F


<400>    41
gtcgtggagt ctactggtgt cttcac                                           26


<210>    42
<211>    28
<212>    DNA
<213>    Artificial Sequence

<220>
<223>    GAPDH-R


<400>    42
gttgtcatat ttctcgtggt tcacaccc                                         28


<210>    43
<211>    27
<212>    DNA

<213>    Artificial Sequence

<220>
<223>    hPDE1a-F


<400>    43
gcttgttcta gctgctgaaa ctgactg                                          27


<210>    44
<211>    22
<212>    DNA
<213>    Artificial Sequence

<220>
<223>    hPDE1a-R


<400>    44
ctgcttccag cacagatgcc gc                                               22


<210>    45
<211>    23
<212>    DNA
<213>    Artificial Sequence

<220>
<223>    hPDE1b-F


<400>    45
gagcttggac tgggagccca aag                                              23


<210>    46
<211>    22
<212>    DNA
<213>    Artificial Sequence

<220>
<223>    hPDE1b-R


<400>    46
gctgacttca gctccagggg tg                                               22


<210>    47
<211>    26
<212>    DNA
<213>    Artificial Sequence

<220>
<223>    hPDE1c-F


<400>    47
gacagcccaa actatctcag cttcac                                           26


<210>    48

<211>    26
<212>    DNA
<213>    Artificial Sequence

<220>
<223>    hPDE1c-R


<400>    48
gatctaccac tgaagcttcc cctctc                                          26


<210>    49
<211>    23
<212>    DNA
<213>    Artificial Sequence

<220>
<223>    hPDE3a-F


<400>    49
gaccacaaac gagggccaag agg                                             23


<210>    50
<211>    25
<212>    DNA
<213>    Artificial Sequence

<220>
<223>    hPDE3a-R


<400>    50
cactgagcga agtgacggga ttcac                                           25


<210>    51
<211>    26
<212>    DNA
<213>    Artificial Sequence

<220>
<223>    hPDE3b-F


<400>    51
ctgcagccag ttactatggc agttgc                                          26


<210>    52
<211>    26
<212>    DNA
<213>    Artificial Sequence

<220>
<223>    hPDE3b-R


<400>    52
ggcttggatc agtcagaaga tctgac                                          26

<210>    53
<211>    23
<212>    DNA
<213>    Artificial Sequence

<220>
<223>    hPDE4a-F


<400>    53
caggcgcttc gaggcagaga atg                                                23


<210>    54
<211>    23
<212>    DNA
<213>    Artificial Sequence

<220>
<223>    hPDE4a-R


<400>    54
cctcgctggt gaccgatgag ttc                                                23


<210>    55
<211>    27
<212>    DNA
<213>    Artificial Sequence

<220>
<223>    hPDE4b-F


<400>    55
gcctttgaca acgcttccaa gcattgc                                            27


<210>    56
<211>    27
<212>    DNA
<213>    Artificial Sequence

<220>
<223>    hPDE4b-R


<400>    56
gctcgcttgg aagagaagag tttctcg                                            27


<210>    57
<211>    22
<212>    DNA
<213>    Artificial Sequence

<220>
<223>    hPDE5a-F


<400>    57

ccagcagcag aagcagcagc ag                                                22

<210>    58
<211>    27
<212>    DNA
<213>    Artificial Sequence

<220>
<223>    hPDE5a-R

<400>    58
cagaggcaga gattttcctg gttggtg                                           27

<210>    59
<211>    23
<212>    DNA
<213>    Artificial Sequence

<220>
<223>    hPDE7a-F

<400>    59
ggaagtgtgt taccagctgc cgg                                               23

<210>    60
<211>    26
<212>    DNA
<213>    Artificial Sequence

<220>
<223>    hPDE7a-R

<400>    60
ggctccttac acgtacatct cctagc                                            26

<210>    61
<211>    26
<212>    DNA
<213>    Artificial Sequence

<220>
<223>    hPDE7b-F

<400>    61
caccgctcag agatttcacg gcattc                                            26

<210>    62
<211>    26
<212>    DNA
<213>    Artificial Sequence

<220>
<223>    hPDE7b-R

<400>    62
cttggtgcca atctcccctg agtatg                                          26


<210>    63
<211>    24
<212>    DNA
<213>    Artificial Sequence

<220>
<223>    hPDE10a-F


<400>    63
gagtgcttcc tgagccccag tttg                                            24


<210>    64
<211>    23
<212>    DNA
<213>    Artificial Sequence

<220>
<223>    hPDE10a-R


<400>    64
gctggttgtc tcctcctgtg tcc                                             23


<210>    65
<211>    22
<212>    DNA
<213>    Artificial Sequence

<220>
<223>    hGAPDH-F


<400>    65
ggcaccgtca aggctgagaa cg                                              22


<210>    66
<211>    23
<212>    DNA
<213>    Artificial Sequence

<220>
<223>    hGAPDH-R


<400>    66
gcagaggggg cagagatgat gac                                             23

<110>    THE ASAN FOUNDATION
         University of Ulsan Foundation For Industry Cooperation

<120>    A Composition for Prevention or Treatment of Neurodegenerative
         disease

<130>    MPCT19-078

<150>    KR 10-2018-0071564
<151>    2018-06-21

<150>    KR 10-2019-0074410
<151>    2019-06-21

<160>    66

<170>    KoPatentIn 3.0

<210>    1
<211>    25
<212>    DNA
<213>    Artificial Sequence

<220>
<223>    PDE1A-F


<400>    1
gaagcagctg gagaaaggtg atgtc                                              25


<210>    2
<211>    25
<212>    DNA
<213>    Artificial Sequence

<220>
<223>    PDE1A-R


<400>    2
cctttccaca aaaatcccag cctgg                                              25


<210>    3
<211>    25
<212>    DNA
<213>    Artificial Sequence

<220>
<223>    PDE1B-F


<400>    3
gcgctacatg gtgaagcagt tggag                                              25


<210>    4
<211>    22
<212>    DNA
<213>    Artificial Sequence

<220>

<223>    PDE1B-R

<400>    4
ccacgaagat cccagcctgc ac                                                22

<210>    5
<211>    24
<212>    DNA
<213>    Artificial Sequence

<220>
<223>    PDE1C-F

<400>    5
cggctgccat ccatgactat gaac                                              24

<210>    6
<211>    24
<212>    DNA
<213>    Artificial Sequence

<220>
<223>    PDE1C-R

<400>    6
gttcgaaact ccctccagtc atcc                                              24

<210>    7
<211>    23
<212>    DNA
<213>    Artificial Sequence

<220>
<223>    PDE2A-F

<400>    7
ggtcataccc ctgctggaca agg                                               23

<210>    8
<211>    25
<212>    DNA
<213>    Artificial Sequence

<220>
<223>    PDE2A-R

<400>    8
cagttgcagg atctttcggt catgg                                             25

<210>    9
<211>    25
<212>    DNA
<213>    Artificial Sequence

<220>
<223>    PDE3a F

<400>    9
actttcatgc tttggagatc ggata                                          25

<210>    10
<211>    22
<212>    DNA
<213>    Artificial Sequence

<220>
<223>    PDE3a R

<400>    10
aggaatcggc tgtgttgtga ga                                             22

<210>    11
<211>    24
<212>    DNA
<213>    Artificial Sequence

<220>
<223>    PDE3B-F

<400>    11
ctctgggact gggacttgaa gcag                                           24

<210>    12
<211>    26
<212>    DNA
<213>    Artificial Sequence

<220>
<223>    PDE3B-R

<400>    12
ctttggccta caggaacctg agaaag                                         26

<210>    13
<211>    22
<212>    DNA
<213>    Artificial Sequence

<220>
<223>    PDE4A-F

<400>    13
gcttggaggc agaaaatggg cc                                             22

<210>    14
<211>    20

<212>    DNA
<213>    Artificial Sequence

<220>
<223>    PDE4A-R


<400>    14
cggagactgg ccagcacctg                                                    20


<210>    15
<211>    25
<212>    DNA
<213>    Artificial Sequence

<220>
<223>    PDE4B-F


<400>    15
gtggaaaatg gcccttctcc aggtc                                              25


<210>    16
<211>    27
<212>    DNA
<213>    Artificial Sequence

<220>
<223>    PDE4B-R


<400>    16
ggcaaaagga gtgacaatca ggtcatc                                            27


<210>    17
<211>    26
<212>    DNA
<213>    Artificial Sequence

<220>
<223>    PDE5A-F


<400>    17
cgcctctttg atgttgctga aggttc                                             26


<210>    18
<211>    26
<212>    DNA
<213>    Artificial Sequence

<220>
<223>    PDE5A-R


<400>    18
cctgatttct tgttgatggc ctgagc                                             26

<210>    19
<211>    24
<212>    DNA
<213>    Artificial Sequence

<220>
<223>    PDE6A-F


<400>    19
gatgtgtggc cagttctgat gggc                                              24


<210>    20
<211>    25
<212>    DNA
<213>    Artificial Sequence

<220>
<223>    PDE6A-R


<400>    20
catccagagg ctctttctgg aattc                                             25


<210>    21
<211>    25
<212>    DNA
<213>    Artificial Sequence

<220>
<223>    PDE6B-F


<400>    21
gataaacgtg caggatgtgg cagag                                             25


<210>    22
<211>    25
<212>    DNA
<213>    Artificial Sequence

<220>
<223>    PDE6B-R


<400>    22
cacagttgtg gaggtagctt aggtg                                             25


<210>    23
<211>    25
<212>    DNA
<213>    Artificial Sequence

<220>
<223>    PDE6C-F


<400>    23
ccggagaagc cagatcctta tgtgg                                             25

<210>    24
<211>    24
<212>    DNA
<213>    Artificial Sequence

<220>
<223>    PDE6C-R


<400>    24
cctgccatct ggagtctttg gtcc                                              24


<210>    25
<211>    26
<212>    DNA
<213>    Artificial Sequence

<220>
<223>    PDE6D-F


<400>    25
gatgaacctt cgggatgccg aaacag                                            26


<210>    26
<211>    26
<212>    DNA
<213>    Artificial Sequence

<220>
<223>    PDE6D-R


<400>    26
gccaggtgtt tgtggagtta gggatc                                            26


<210>    27
<211>    22
<212>    DNA
<213>    Artificial Sequence

<220>
<223>    PDE7A-F


<400>    27
cagctctcgc agagacgtgg ag                                                22


<210>    28
<211>    26
<212>    DNA
<213>    Artificial Sequence

<220>
<223>    PDE7A-R

<400>    28
ccagcataca cttggcttgt ccattg                                            26


<210>    29
<211>    27
<212>    DNA
<213>    Artificial Sequence

<220>
<223>    PDE7B-F


<400>    29
gttgtgtcac ctcttcaact cccatgg                                           27


<210>    30
<211>    23
<212>    DNA
<213>    Artificial Sequence

<220>
<223>    PDE7B-R


<400>    30
gcatgatgtc cagaggtgtg agg                                               23


<210>    31
<211>    24
<212>    DNA
<213>    Artificial Sequence

<220>
<223>    PDE8A-F


<400>    31
gtcaccaagg aggtgcagac tgtc                                              24


<210>    32
<211>    26
<212>    DNA
<213>    Artificial Sequence

<220>
<223>    PDE8A-R


<400>    32
gagttacaag ccctgagttt cagctg                                            26


<210>    33
<211>    27
<212>    DNA
<213>    Artificial Sequence

<220>
<223>    PDE8B-F

<400>    33
cagtgtttac agcgttagac cactgtc                                          27


<210>    34
<211>    27
<212>    DNA
<213>    Artificial Sequence

<220>
<223>    PDE8B-R


<400>    34
cgaatgaggc tctgtctgag agttgtc                                          27


<210>    35
<211>    22
<212>    DNA
<213>    Artificial Sequence

<220>
<223>    PDE9A-F


<400>    35
gacacctcga cgtgatgtcc cc                                               22


<210>    36
<211>    25
<212>    DNA
<213>    Artificial Sequence

<220>
<223>    PDE9A-R


<400>    36
catctgtgtc acacagaagc agtgc                                            25


<210>    37
<211>    26
<212>    DNA
<213>    Artificial Sequence

<220>
<223>    PDE10A-F


<400>    37
gggattgctg gtcaagtggc aagaac                                           26


<210>    38
<211>    27
<212>    DNA
<213>    Artificial Sequence

<220>
<223>    PDE10A-R


<400>    38
gcttctccat ggtaaccctg tagatgc                                          27


<210>    39
<211>    22
<212>    DNA
<213>    Artificial Sequence

<220>
<223>    PDE11A-F


<400>    39
gctggaggtg gtcaatgacc tc                                               22


<210>    40
<211>    23
<212>    DNA
<213>    Artificial Sequence

<220>
<223>    PDE11A-R


<400>    40
ctgtcgaagc aaccagctca gcg                                              23


<210>    41
<211>    26
<212>    DNA
<213>    Artificial Sequence

<220>
<223>    GAPDH-F


<400>    41
gtcgtggagt ctactggtgt cttcac                                           26


<210>    42
<211>    28
<212>    DNA
<213>    Artificial Sequence

<220>
<223>    GAPDH-R


<400>    42
gttgtcatat ttctcgtggt tcacaccc                                         28


<210>    43
<211>    27
<212>    DNA

<213>    Artificial Sequence

<220>
<223>    hPDE1a-F


<400>    43
gcttgttcta gctgctgaaa ctgactg                                          27


<210>    44
<211>    22
<212>    DNA
<213>    Artificial Sequence

<220>
<223>    hPDE1a-R


<400>    44
ctgcttccag cacagatgcc gc                                               22


<210>    45
<211>    23
<212>    DNA
<213>    Artificial Sequence

<220>
<223>    hPDE1b-F


<400>    45
gagcttggac tgggagccca aag                                              23


<210>    46
<211>    22
<212>    DNA
<213>    Artificial Sequence

<220>
<223>    hPDE1b-R


<400>    46
gctgacttca gctccagggg tg                                               22


<210>    47
<211>    26
<212>    DNA
<213>    Artificial Sequence

<220>
<223>    hPDE1c-F


<400>    47
gacagcccaa actatctcag cttcac                                           26


<210>    48

<211>    26
<212>    DNA
<213>    Artificial Sequence

<220>
<223>    hPDE1c-R


<400>    48
gatctaccac tgaagcttcc cctctc                                          26


<210>    49
<211>    23
<212>    DNA
<213>    Artificial Sequence

<220>
<223>    hPDE3a-F


<400>    49
gaccacaaac gagggccaag agg                                             23


<210>    50
<211>    25
<212>    DNA
<213>    Artificial Sequence

<220>
<223>    hPDE3a-R


<400>    50
cactgagcga agtgacggga ttcac                                           25


<210>    51
<211>    26
<212>    DNA
<213>    Artificial Sequence

<220>
<223>    hPDE3b-F


<400>    51
ctgcagccag ttactatggc agttgc                                          26


<210>    52
<211>    26
<212>    DNA
<213>    Artificial Sequence

<220>
<223>    hPDE3b-R


<400>    52
ggcttggatc agtcagaaga tctgac                                          26

<210>    53
<211>    23
<212>    DNA
<213>    Artificial Sequence

<220>
<223>    hPDE4a-F


<400>    53
caggcgcttc gaggcagaga atg                                                23


<210>    54
<211>    23
<212>    DNA
<213>    Artificial Sequence

<220>
<223>    hPDE4a-R


<400>    54
cctcgctggt gaccgatgag ttc                                                23


<210>    55
<211>    27
<212>    DNA
<213>    Artificial Sequence

<220>
<223>    hPDE4b-F


<400>    55
gcctttgaca acgcttccaa gcattgc                                            27


<210>    56
<211>    27
<212>    DNA
<213>    Artificial Sequence

<220>
<223>    hPDE4b-R


<400>    56
gctcgcttgg aagagaagag tttctcg                                            27


<210>    57
<211>    22
<212>    DNA
<213>    Artificial Sequence

<220>
<223>    hPDE5a-F


<400>    57

ccagcagcag aagcagcagc ag                                                22

<210>    58
<211>    27
<212>    DNA
<213>    Artificial Sequence

<220>
<223>    hPDE5a-R

<400>    58
cagaggcaga gattttcctg gttggtg                                           27

<210>    59
<211>    23
<212>    DNA
<213>    Artificial Sequence

<220>
<223>    hPDE7a-F

<400>    59
ggaagtgtgt taccagctgc cgg                                               23

<210>    60
<211>    26
<212>    DNA
<213>    Artificial Sequence

<220>
<223>    hPDE7a-R

<400>    60
ggctccttac acgtacatct cctagc                                            26

<210>    61
<211>    26
<212>    DNA
<213>    Artificial Sequence

<220>
<223>    hPDE7b-F

<400>    61
caccgctcag agatttcacg gcattc                                            26

<210>    62
<211>    26
<212>    DNA
<213>    Artificial Sequence

<220>
<223>    hPDE7b-R

<400>    62
cttggtgcca atctcccctg agtatg                                          26


<210>    63
<211>    24
<212>    DNA
<213>    Artificial Sequence

<220>
<223>    hPDE10a-F


<400>    63
gagtgcttcc tgagccccag tttg                                            24


<210>    64
<211>    23
<212>    DNA
<213>    Artificial Sequence

<220>
<223>    hPDE10a-R


<400>    64
gctggttgtc tcctcctgtg tcc                                             23


<210>    65
<211>    22
<212>    DNA
<213>    Artificial Sequence

<220>
<223>    hGAPDH-F


<400>    65
ggcaccgtca aggctgagaa cg                                              22


<210>    66
<211>    23
<212>    DNA
<213>    Artificial Sequence

<220>
<223>    hGAPDH-R


<400>    66
gcagaggggg cagagatgat gac                                             23